# EUROPEAN PATENT APPLICATION

(11) **EP 1 149 909 A1**
(43) Date of publication of application: **31.10.2001**
(21) Application number: 00109270.9
(22) Date of filing: 28.04.2000
(51) Int. Cl.: C12N 15/31, C07K 14/245, C12N 9/04, C07K 1/113, A61K 39/02, A61K 48/00

(54) **Methods for regulating protein conformation using molecular chaperones**

(71) Applicant: Roche Diagnostics GmbH, 68305 Mannheim (DE); Bukau, Bernd, Prof. Dr., 79104 Freiburg (DE)
(72) Inventor: Bukau, Bernd, Prof., 79104 Freiburg (DE); Goloubinoff, Pierre, Dr., 93855 Jerusalem (IL)

(57) **Abstract**

The present invention provides a method for regulating the confirmation of proteins using molecular chaperones. The chaperones of the present invention are applicable to a variety of protein conformational problems including misfolding and aggregation. A molecular chaperone system of the present invention includes the DnaK system and ClpB which provides a particularly effective tool for regulating protein conformation. A further subject of the present invention is a cochaperone ClpS.

### Annex to the application documents - subsequently filed sequences listing

SEQUENCE LISTING

<110> Roche Diagnostics GmbH

<120> Methods for regulating protein conformation using
      molecular chaperones

<130> 5392/OA/EP

<140>
<141>

<160> 9

<170> PatentIn Ver. 2.1

<210> 1
<211> 318
<212> DNA
<213> E. coli

<220>
<221> CDS
<222> (1)..(318)

<400> 1
atg ggt aaa acg aac gac tgg ctg gac ttt gat caa ctg gcg gaa gaa     48
Met Gly Lys Thr Asn Asp Trp Leu Asp Phe Asp Gln Leu Ala Glu Glu
  1               5                  10                  15

aaa gtt cgc gac gcg cta aaa ccg cca tct atg tat aaa gtg ata tta     96
Lys Val Arg Asp Ala Leu Lys Pro Pro Ser Met Tyr Lys Val Ile Leu
             20                  25                  30

gtc aat gat gat tac act ccg atg gag ttt gtt att gac gtg tta caa     144
Val Asn Asp Asp Tyr Thr Pro Met Glu Phe Val Ile Asp Val Leu Gln
         35                  40                  45

aaa ttc ttt tct tat gat gta gaa cgt gca acg caa ttg atg ctc gct     192
Lys Phe Phe Ser Tyr Asp Val Glu Arg Ala Thr Gln Leu Met Leu Ala
     50                  55                  60

gtt cac tac cag ggg aag gcc att tgc gga gtc ttt acc gcc gag gtt     240
Val His Tyr Gln Gly Lys Ala Ile Cys Gly Val Phe Thr Ala Glu Val
 65                  70                  75                  80

gca gaa acc aaa gtg gcg atg gtg aac aag tac gcg agg gag aat gag     288
Ala Glu Thr Lys Val Ala Met Val Asn Lys Tyr Ala Arg Glu Asn Glu
                 85                  90                  95

cat cca ttg ctg tgt acg cta gaa aaa gcc [?]                         319
His Pro Leu Leu Cys Thr Leu Glu Lys Ala
            100                 105


<210> 2
<211> 106
<212> PRT
<213> E. coli

<400> 2
Met Gly Lys Thr Asn Asp Trp Leu Asp Phe Asp Gln Leu Ala Glu Glu
1               5                   10                  15

Lys Val Arg Asp Ala Leu Lys Pro Pro Ser Met Tyr Lys Val Ile Leu
            20                  25                  30

Val Asn Asp Asp Tyr Thr Pro Met Glu Phe Val Ile Asp Val Leu Gln
        35                  40                  45

Lys Phe Phe Ser Tyr Asp Val Glu Arg Ala Thr Gln Leu Met Leu Ala
    50                  55                  60

Val His Tyr Gln Gly Lys Ala Ile Cys Gly Val Phe Thr Ala Glu Val
65                  70                  75                  80

Ala Glu Thr Lys Val Ala Met Val Asn Lys Tyr Ala Arg Glu Asn Glu
                85                  90                  95

His Pro Leu Leu Cys Thr Leu Glu Lys Ala
            100                 105


<210> 3
<211> 123
<212> PRT
<213> A. thaliana

<400> 3
Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa Xaa Xaa Xaa Xaa Xaa Xaa Xaa Xaa Xaa Xaa Xaa Xaa
            20                  25                  30

Phe Arg Glu Ser Lys Glu Ile Ala Pro Thr Tyr Arg Val Ile Leu His
        35                  40                  45

Asn Asp Asn Phe Asn Lys Arg Glu Tyr Val Val Gln Val Leu Met Lys
    50                  55                  60

Val Ile Pro Gly Met Thr Val Asp Asn Ala Val Asn Ile Met Gln Glu
65                  70                  75                  80

Ala His Ile Asn Gly Leu Ala Val Val Ile Val Cys Ala Gln Ala Asp
                85                  90                  95

Ala Glu Gln His Cys Met Xaa Cys Ala Val Thr Ala Phe Ser Val Leu
            100                 105                 110

Leu Asn Leu Met Val Glu Ala Ala Glu Thr Asn
        115                 120


<210> 4
<211> 122
<212> PRT
<213> G. max

<400> 4
Met Val Val Leu Ala Thr Gly Pro Gly Ile Gly Gly Gly Val Leu Glu
1               5                   10                  15

Lys Pro Val Ile Glu Lys Val Thr Pro Gly Arg Glu Ser Glu Phe Asp
            20                  25                  30

Leu Lys Lys Ser Arg Lys Ser Ala Pro Pro Tyr Arg Val Ile Leu His
        35                  40                  45

Asn Asp Asn Phe Asn Lys Arg Glu Tyr Val Val Gln Val Leu Met Lys
    50                  55                  60

Val Ile Pro Gly Met Thr Leu Asp Asn Ala Val Asn Ile Met Gln Glu
65                  70                  75                  80

Ala His Tyr Asn Gly Leu Ser Val Val Ile Ile Cys Asp Gln Ala Asp
                85                  90                  95

Ala Glu Xaa Xaa Xaa Xaa Xaa Xaa Xaa Xaa Xaa Xaa Xaa Xaa Xaa Xaa
            100                 105                 110

Xaa Xaa Ile Thr Ala Xaa Xaa Xaa Xaa Cys
        115                 120


<210> 5
<211> 122
<212> PRT
<213> Z. mays

<400> 5
Met Ala Leu Ser Ala Ala Ala Pro Gly Lys Gly Gly Gly Val Leu Asp
1               5                   10                  15

Arg Pro Val Xaa Glu Lys Thr Thr Pro Gly Arg Gln Ser Glu Phe Asp
            20                  25                  30

Val Lys Lys Ser Arg Lys Met Ser Pro Pro Tyr Arg Val Ile Leu His
        35                  40                  45

Asn Asp Asn Tyr Asn Arg Arg Glu Tyr Val Val Lys Val Leu Met Lys
    50                  55                  60

Val Ile Pro Gly Met Thr Val Asp Asn Ala Val Asn Ile Met Gln Glu
65                  70                  75                  80

Ala His Val Asn Gly Leu Ser Val Val Ile Val Cys Ser Gln Ser Glu
                85                  90                  95

Ala Glu Glu His Cys Thr Ser Xaa Xaa Leu Arg Gly Asn Gly Leu Arg
            100                 105                 110

Ser Ser Ile Glu Pro Ala Ser Gly Gly Cys
        115                 120


<210> 6

<211> 123
<212> PRT
<213> Synechcystis no. 1

<400> 6
Met Ile Ser Asn Ala Ala Thr Ser Xaa Xaa Xaa Xaa Xaa Xaa Xaa Xaa
1               5                   10                  15

Xaa Xaa Xaa Xaa Xaa Xaa Xaa Thr Pro Asp Arg Leu Thr Ser Xaa Xaa
            20                  25                  30

Xaa Xaa Thr Val Arg Lys Thr Tyr Pro Asn Phe Lys Val Ile Val Leu
        35                  40                  45

Asn Asp Asp Phe Asn Thr Phe Gln His Val Ser Asp Cys Leu Leu Lys
    50                  55                  60

Tyr Ile Pro Gly Met Thr Gly Asp Arg Ala Trp Glu Leu Thr Asn Gln
65                  70                  75                  80

Val His Phe Asp Gly Leu Ala Ile Val Trp Val Gly Pro Gln Glu Gln
                85                  90                  95

Ala Glu Leu Tyr His Gln Xaa Xaa Gln Leu Arg Arg Glu Gly Leu Xaa
            100                 105                 110

Xaa Xaa Xaa Thr Met Ala Pro Leu Glu Lys Ala
        115                 120


<210> 7
<211> 120
<212> PRT
<213> Synechcystis no. 2

<400> 7
Met Met Xaa Xaa Xaa Xaa Xaa Xaa Xaa Xaa Ala Thr Glu Val Leu Asn
1               5                   10                  15

Lys Pro Ser Asn Ser Thr Ile Xaa Xaa Xaa Xaa Xaa Xaa Xaa Xaa Xaa
            20                  25                  30

Xaa Xaa Xaa Xaa Arg Lys His Ala Pro Arg Tyr Arg Val Leu Leu His
        35                  40                  45

Asn Asp Asp Phe Asn Ser Met Glu His Val Val Gln Thr Leu Ile Gln
    50                  55                  60

Thr Val Ala Gly Met Thr Gln Pro Gln Ala Val Asp Ile Met Met Glu
65                  70                  75                  80

Ala His Phe Asn Gly Met Ser Leu Val Ile Thr Cys Glu Leu Glu His
                85                  90                  95

Ala Glu Phe Tyr Cys Glu Thr Xaa Xaa Leu Arg Ser His Gly Leu Ser
            100                 105                 110

Ser Thr Ile Glu Pro Asp Glu Xaa
        115                 120

<210> 8
<211> 122
<212> PRT
<213> E. coli

<400> 8
Met Xaa Xaa Xaa Xaa Xaa Xaa Xaa Xaa Xaa Xaa Xaa Xaa Xaa Xaa Xaa
1               5                   10                  15

Gly Lys Thr Asn Asp Trp Leu Asp Phe Asp Gln Leu Ala Glu Glu Lys
            20                  25                  30

Val Arg Asp Ala Leu Lys Pro Pro Ser Met Tyr Lys Val Ile Leu Val
        35                  40                  45

Asn Asp Asp Tyr Thr Pro Met Glu Phe Val Ile Asp Val Leu Gln Lys
    50                  55                  60

Phe Phe Ser Xaa Tyr Asp Val Glu Arg Ala Thr Gln Leu Met Leu Ala
65                  70                  75                  80

Val His Tyr Gln Gly Lys Ala Ile Cys Gly Val Phe Thr Ala Glu Val
                85                  90                  95

Ala Glu Thr Lys Val Ala Met Val Asn Lys Tyr Ala Arg Glu Asn Glu
            100                 105                 110

His Pro Leu Leu Cys Thr Leu Glu Lys Ala
        115                 120


<210> 9
<211> 123
<212> PRT
<213> H. pylori

<400> 9
Met Xaa Xaa Xaa Xaa Xaa Xaa Xaa Xaa Xaa Xaa Xaa Xaa Xaa Xaa Xaa
1               5                   10                  15

Lys Met Tyr Asn Xaa Xaa Xaa Xaa Xaa Xaa Xaa Xaa Xaa Xaa Xaa Xaa
            20                  25                  30

Xaa Xaa Xaa Xaa Ile Pro Thr Pro Thr Met Ala Gln Val Ile Met Val
        35                  40                  45

Asp Asp Pro Ile Thr Thr Thr Glu Phe Val Ile Ser Ala Leu Arg Asp
    50                  55                  60

Phe Phe Asp Xaa Lys Ser Leu Glu Glu Ala Lys Ala Leu Thr Ser Ser
65                  70                  75                  80

Ile His Arg Asp Gly Glu Gly Val Cys Gly Val Tyr Pro Tyr Asp Ile
                85                  90                  95

Ala Arg His Arg Ala Ala Trp Val Arg Asp Lys Ala Lys Ala Leu Glu
            100                 105                 110

Phe Pro Leu Lys Leu Leu Val Glu Glu Ile Lys
        115                 120

## Description

The invention relates to the use of molecular chaperones for regulating the conformation of proteins, to novel chaperone systems and cochaperones. All patents, published patent applications and other references cited throughout this specification are hereby incorporated by reference in their entireties.

The functional activity of proteins requires a balance between structural stability and flexibility, achieved by low conformational stability of the folded proteins. Implicit to this balance is that correct and incorrect folding as well as native and nonnative structures are frequently separated by only small energy barriers (Jaenicke and Seckler, 1999), and subtle changes in the amino acid sequence or the folding milieu may have dramatic effects on folding and structural integrity of proteins. Protein misfolding is indeed the major damaging consequence of a variety of stress situations including heat and oxidative stress (Bukau, 1999). Misfolded proteins generally expose hydrophobic surfaces and are thus prone to intermolecular aggregation, a process long considered irreversible *in vivo*. Protein aggregation has biomedical importance, and has been suggested as playing a causative role in several pathophysiological states including amyloidosis and prion diseases (Horwich and Weissman, 1997; Lindquist, 1997; Prusiner, 1997; Thomas *et al., 1995;* Wetzel, 1996). Furthermore, aggregation with concomitant formation of inclusion bodies is a frequent fate of recombinant proteins overproduced in host cells and thus is of considerable importance in biotechnology (Lilie *et al.,* 1998; Mitraki and King, 1989).

One solution for protein misfolding and aggregation involves molecular chaperones which can prevent aggregation of misfolded proteins, and either assist in protein refolding or cooperate with proteases to promote degradation of an aggregate (Bukau, 1999; Hard, 1996; Morimoto *et al.*, 1994). Many chaperones, including Hsp90 (HtpG) and small HSPs (IbpA and IbpB), form a functional network and act as holders to prevent aggregation of misfolded proteins (E. *coli* homologs are given in parentheses). Other chaperones, in particular the Hsp60 (GroEL) and Hsp70 (DnaK) heat shock proteins with their respective co-chaperones (being GroES for GroEL, and DnaJ and GrpE in case of DnaK), act as folders which not only prevent aggregation but also assist refolding (Buchberger *et al.*, 1996; Ehrnsperger *et al.*, 1998; Freeman *et al.*, 1996; Johnson and Craig, 1997; Langer *et al.*, 1992; Veinger *et al.*, 1998). Yet other chaperones, in particular the Trigger Factor of eubacteria, associate with ribosomes and interact with nascent polypeptide chains during biosynthesis. The role of Trigger Factor in protein folding in vivo is not documented in the literature.

An intrinsic feature of the activity of chaperones is their ability to interact repeatedly with protein folding intermediates, allowing the substrates to dissociate from the chaperone and to either fold to native state or rebind to chaperones of the same or another type. These cycles of substrate interaction are regulated for many chaperones by ATP binding and hydrolysis. It has been demonstrated that cells have only limited capacity of chaperone activity (Craig and Gross, 1991; Tatsuta et al., 1998; Tomoyasu et al., 1998), suggesting that the assistance of protein folding processes by chaperones may become rate-limiting during overproduction of recombinant proteins and under pathophysiological stress conditions of cells.

A largely unexplored aspect of chaperone activity is the potential to dissolve pre-existing protein aggregates and to mediate refolding. The DnaK chaperone of *E. coli*, in association with its co-chaperones, has the ability to disaggregate slowly heat denatured RNA polymerase of *E. coli* (Skowyra et al., 1990; Ziemienowicz et al., 1993). However, this disaggregating activity appears to be atypical for DnaK and other members of the Hsp70 family because in all other reported cases, members of the Hsp70 family demonstrated no or only slight disaggregation activity (e.g., Schroder et al., 1993). In contrast, the S. *cerevisiae* member of the Hsp100 family, the Hsp104 heat shock protein, has been shown to disaggregate heat denatured firefly luciferase and β-galactosidase, thereby allowing the Hsp70 chaperone system to refold these enzymes (Glover and Lindquist, 1998; Parsell et al., 1994).

The published evidence demonstrates the high potential of the cellular system of molecular chaperones to assist a large variety of protein folding processes. However, there is only limited published knowledge on the particular role of individual bacterial chaperones in protecting proteins from aggregation and in disaggregation and/or refolding of proteins in vivo, and no information on a general activity of bacterial chaperones in disaggregating and refolding of proteins.

The present invention is directed to the use of a class of cellular proteins, collectively termed molecular chaperones, to mediate disaggregation and refolding of aggregated proteins as well as refolding of misfolded proteins. The invention further features a combination of two chaperone systems of E. *coli*, ClpB (in Hsp100 protein) and the DnaK system (including DnaK, a Hsp70 protein), to mediate the efficient disaggregation and refolding of a broad spectrum of aggregated proteins. This combination of chaperones is useful for preventing and reversing formation of aggregates and inclusion bodies of recombinant proteins upon overproduction in *E. coli* and other host cells and for increasing the production yields of soluble and active recombinant proteins. These chaperones can be employed *in vivo*, in particular but not 5exclusively in *E. coli*, and *in vitro*. The invention also provides a method for treating diseases caused, for example, by misfolding and aggregation of a protein using the molecular chaperone system disclosed herein.

Thus, in one aspect, the invention is a method for regulating the conformation of a target protein by contacting the target protein with a molecular chaperone system having a first protein of at least one Hsp100 protein or a homolog thereof and a second protein of at least one Hsp70 protein or a homolog thereof to regulate the conformation of the target protein.

The invention provides a system of recombinant expression vectors. In accordance with one embodiment, a single recombinant expression vector encodes at least one Hsp100 protein or homologue thereof and at least one Hsp70 protein or homologue thereof. The expression vector can further encode one or more chaperones for one or both of the Hsp100 and Hsp70 proteins, as well as protein of interest. In another embodiment, a single vector is used to express each protein component of the molecular chaperone system.

In another embodiment, the invention provides a host cell transformed with a vector or vector system. In a preferred embodiment, the host cell is engineered to express recombinantly a protein of interest in addition to the molecular chaperone system described above. In practicing the method of the invention, for example, by using a host cell so engineered, an inhibition of the aggregation and misfolding of the protein of interest can be achieved, thereby increasing the yield of the active conformation of the protein of interest as compared to a cell where the molecular chaperone system is not recombinantly expressed.

Another aspect of the invention is a method for disaggregating an aggregated protein *in vitro* by contacting the aggregated protein with a molecular chaperone system having at least one Hsp100 protein or a homolog thereof and at least one Hsp70 protein or a homolog thereof to disaggregate the aggregated protein. In another embodiment, the invention provides a method for increasing the solubility of a recombinantly expressed protein by coexpressing a molecular chaperone system having at least one Hsp100 protein or a homolog thereof and at least one Hsp70 protein or a homolog thereof to increase the solubility of the recombinantly expressed protein.

In another embodiment, the invention is a method for preventing cell death caused by misfolding or aggregation of a protein which comprises administering a therapeutically effective amount of a molecular chaperone system having at least one Hsp100 protein or a homolog thereof and at least one Hsp70 protein or a homolog thereof to prevent the misfolding or aggregation of the protein.

The invention also provides a method of treating a disease in an animal caused by misfolding and aggregation of a protein by administering a therapeutically effective dose of a molecular chaperone system having at least one Hsp100 protein or a homolog thereof and at least one Hsp70 protein or a homolog thereof to thereby treat the disease. The invention also provides gene therapies for treating disease using polynucleic acid molecules encoding the molecular chaperone systems disclosed herein.

A further embodiment of the invention is a pharmaceutical composition comprising an effective dose of a molecular chaperone system comprising at least one Hsp100 protein or a homolog thereof and at least one Hsp70 protein or a homolog thereof, and a pharmaceutically acceptable carrier.

Another embodiment of the invention is a method for increasing the folding efficiency of a protein *in vitro* by adding Trigger Factor, with or without at least one Hsp70 protein or homolog, to an *in vitro* protein synthesis system to increase the folding efficiency of proteins *in vitro*.

In another aspect, the invention is an isolated polynucleotide encoding a ClpS protein and derivatives thereof capable of regulating Hsp100 proteins or homologs thereof in disaggregation. In one embodiment, the invention provides polypeptides such as ClpS and derivatives thereof capable of regulating Hsp100 homologs in disaggregation. A further embodiment includes methods of regulating a Hsp100 protein or homolog thereof in disaggregation.

The invention also provides a molecular chaperone system to regulate protein conformation comprising a first protein of at least one Hsp100 protein or a homolog thereof and a second protein of at least one Hsp70 protein or a homolog thereof.

### Brief Description of the Drawings

Fig. 1 depicts the prevention of heat-induced protein aggregation by cytosolic chaperone systems in cell extracts.

Fig. 2 shows protein aggregation in wild type and *DnaK* mutant cells after heat-shock to 42°C.

Fig. 3 depicts the aggregation of heat-labile proteins in cell extracts.

Fig. 4 shows the disaggregation of protein aggregates *in vitro* by a molecular chaperone system combining the DnaK system and ClpB as compared to other molecular chaperones.

Fig. 5 depicts gel electrophoresis showing the disaggregation of protein aggregates *in vitro* by DnaK, DnaJ, GrpE and ClpB.

Fig. 6 shows ClpB and DnaK-mediated disaggregation and reactivation of heat-inactivated Malate Dehydrogenase.

Fig. 7 shows the disaggregation of aggregated proteins after heat-shock *in vivo* in E. *coli* wild type and *dnak* and *clpB* null mutant cells.

Fig. 8 shows disaggregation of protein aggregates by DnaK, DnaJ and ClpB *in vivo*.

Figs. 9A and 9B is a schematic representation of the chaperone overproduction system in E. *coli*.

Figs. 10A and 10B show the synthetic lethality of DnaK/DnaJ depleted *Atig::kan* cells.

Figs. 11A and 11B depict the insolubility of pre-existing and newly synthesized proteins.

Fig. 12 depicts the nucleic acid sequence (SEQ ID NO:1) and the deduced amino acid sequence (SEQ ID NO:2) of YljA protein.

Fig. 13 represents an amino acid sequence alignment of ClpS homologs.

Fig. 14 shows the ClpA/ClpS system reactivates Malate dehydrogenase.

Fig. 15 Overproduction of ClpB and the DnaK system improves heat resistance and luciferase refolding of *rpoH* and wild type cells.

### Detailed Description of the Invention

### Definitions

Before further description of the invention, certain terms employed in the specification, examples and appended claims are for convenience collected here.

"DNA" refers to deoxyribonucleic acid whether single- or doublestranded.

"Complementary DNA" (cDNA) is DNA which has a nucleic acid sequence obtained from reverse transcription of messenger ribonucleic acid (mRNA).

"Recombinant genetic expression" (also recombination or genetic recombination or recombinant expression) refers to the methods by which a nucleic acid molecule encoding a polypeptide or protein of interest is used to transform a host cell so that the host cell can express the polypeptide of interest. A plasmid or vector can be used to introduce a nucleic acid molecule into a host cell. A plasmid or vector can comprise, but need not, in addition to the gene or nucleic acid sequence of interest, a gene that expresses a selectable marker or phenotype and a gene that can control (induce or inhibit) the expression of the gene of interest under certain conditions.

"Protein conformation" refers to the structure of a protein including its folding and function. A normal protein is generally active, i.e., able to perform its normal function. An inactive protein is not able to perform its normal function or is not able to perform its normal function at the normal rate. Protein inactivity can be the result of numerous factors, including misfolding. Misfolded proteins can become aggregated.

"Protein aggregation" is the formation of a fiber, an amorphous clump, an inclusion body, a dimer, trimer or other protein formation of more than one protein wherein at least one protein is misfolded or inactive.

A "soluble protein" is a protein that is not aggregated but may be misfolded.

A "molecular chaperone" is protein that modulates, regulates or assists in protein conformation. A molecular chaperone can cause proteins to fold properly, refold, prevent aggregation or disaggregate proteins. An example of a molecular chaperone is DnaK, although many different kinds of chaperones exist within a species, and homologs between species exist as well.

A "cochaperone" is a protein that improves the ability of the cochaperone to perform its function in protein conformation. Example of cochaperones includes, but are not limited to, DnaJ and GrpE which assist in the function of DnaK.

A "molecular chaperone system" comprises a molecular chaperone and either a cochaperone or a second chaperone. Hsp70 and Hsp100 proteins are described at length in U.S. Patent 5,827,685 to Lindquist. "Homologs," used here generally, of the Hsp70 and Hsp100 proteins exist in various species and can be found, for example, in *E. coli* For example, ClpB is an E. Coli Hsp100 protein homolog and, more specifically, corresponds to Hsp104. DnaK is an E Coli Hsp70 protein. DnaJ and GrpE are *E. coli* DnaK cochaperones and their homologs exist in other pro-caryotes and eucaryotes as well.

The term "family" when referring to the protein and nucleic acid molecules of the invention is intended to mean two or more proteins or nucleic acid molecules having a common structural domain and having sufficient amino acid or nucleotide sequence homology as defined herein. Such family members can be naturally occurring and can be from either the same or different species. For example, a family can contain a first protein of human origin, as well as other, distinct proteins of human origin or alternatively, can contain homologues of non-human origin. Members of a family may also have common functional characteristics.

"DnaK system" refers to DnaK and the cochaperones DnaJ and GrpE.

"The GroEL-GroES system" is an Hsp60 protein (GroEL) and its cochaperone (GroES).

"Protein of interest," "target protein" and "substrate" are used interchangeably and share the same meaning in that they refer to any protein or polypeptide. "Protein of interest" is sometimes used when referring to a recombinantly expressed protein whether *in vivo, in vitro*, isolated or purified but refers to any protein or polypeptide unless further specified. "Target protein" is sometimes used when referring to any protein to be acted upon whether or not recombinantly expressed or synthesized but refers to any protein or polypeptide unless further specified. "Substrate" is sometimes used when referring to any protein or polypeptide to be acted upon but refers to any protein or polypeptide unless further specified.

"A vector system" is a system of recombinant expression vectors. In one instance a single recombinant expression vector can encode one or more proteins. In another instance, the vector can express one, two, three, *etc*. proteins. A host cell can be transformed with one or more vectors of the system. A preferred vector system is a cassette-like plasmid system developed by Bujard and coworkers (Lutz and Bujard, 1997) is used to deploy the recombinant vector system.

"Polypeptide derivatives/protein derivatives" as used herein refer to polypeptide/protein sequences that differ from the sequences described or known in amino acid sequence, or in ways that do not involve sequence, or both, and still preserve the activity of the polypeptide or protein. Derivatives in amino acid sequence are produced when one or more amino acids is substituted with a different natural- amino acid, an amino acid derivative or non-native amino acid. Particularly preferred embodiments include naturally occurring polypeptides or proteins, or biologically active fragments thereof, whose sequences differ from the wild type sequence by one or more conservative amino acid substitutions, which typically have minimal influence on the secondary structure and hydrophobic nature of the protein or peptide. Derivatives may also have sequences which differ by one or more non-conservative amino acid substitutions, deletions or insertions which do not abolish the biological activity of the polypeptide or protein. Conservative substitutions (substituents) typically include the substitution of one amino acid for another with similar characteristics such as substitutions within the following groups: valine, glycine; glycine, alanine; valine, isoleucine; aspartic acid, glutamic acid; asparagine, glutamine; serine, threonine; lysine, arginine; and phenylalanine, tyrosine. The non-polar (hydrophobic) amino acids include alanine, leucine, isoleucine, valine, proline, phenylalanine, tryptophan and methionine. The polar neutral amino acids include glycine, serine, threonine, cysteine, tyrosine, asparagine and glutamine. The positively charged (basic) amino acids include arginine, lysine and histidine. The negatively charged (acidic) amino acids include aspartic acid and glutamic acid.

Other conservative substitutions can be taken from Table 1, and yet others are described by Day-hoffin the Atlas of Protein Sequence and Structure (1988).

**Table 1**

| Conservative Amino Acid Replacements | | |
|---|---|---|
| For Amino Acid | Code | Replace with any of |
| Alanine | A | D-Ala, Gly, beta-Ala, L-Cys,D-Cys |
| Arginine | R | D-Arg, Lys,homo-Arg, D-homo-Arg, Met,D-Met, |
| | | Ile, D-Ile, Orn, D-Orn |
| Asparagine | N | D-Asn,Asp,D-Asp,Glu,D-Glu, Gln,D-Gln |
| Aspartic Acid | D | D-Asp,D-Asn,Asn, Glu,D-Glu, Gln, D-Gln |
| Cysteine | C | D-Cys, S-Me-Cys,Met,D-Met,Thr,D-Thr |
| Glutamine | Q | D-Gln,Asn, D-Asn,Glu,D-Glu,Asp, D-Asp |
| Glutamic Acid | E | D-Glu,D-Asp,Asp, Asn, D-Asn, Gln, D-Gln |
| Glycine | G | Ala, D-Ala,Pro, D-Pro, Beta-Ala, Acp |
| Isoleucine | I | D-Ile, Val, D-Val, Leu, D-Leu, Met, D-Met |
| Leucine | L | D-Leu, Val, D-Val, Met, D-Met |
| Lysine | K | D-Lys,Arg, D-Arg, homo-Arg, D-homo-Arg, Met, D-Met, Ile, D-Ile, Orn, D-Orn |
| Methionine | M | D-Met, S-Me-Cys, Ile, D-Ile, Leu, D-Leu, Val, D Val, Norleu |
| Phenylalanine | F | D-Phe,Tyr, D-Thr,L-Dopa,His,D-His, Trp, D-Trp, Trans 3,4 or 5-phenylproline, cis 3,4 or 5 phenylproline |
| Proline | P | D-Pro, L-I-thioazolidine-4-carboxylic acid, D- or L 1-oxazolidine-4-carboxylic acid |
| Serine | S | D-Ser, Thr, D-Thr, allo-Thr, Met, D-Met, Met(O), D-Met(O), Val, D-Val |
| Threonine | T | D-Thr, Ser, D-Ser, allo-Thr, Met, D-Met, Met(O) D Met(O), Val, D-Val |
| Tyrosine | Y | D-Tyr,Phe, D-Phe, L-Dopa, His,D-His |
| Valine | V | D-Val, Leu,D-Leu,Ile,D-Ile, Met, D-Met |

Other derivatives within the invention are those with modifications which increase peptide stability. Such derivatives may contain, for example, one or more non-peptide bonds (which replace the peptide bonds) in the peptide sequence. Also included are derivatives that include residues other than naturally occurring L-amino acids, such as D-amino acids or non-naturally occurring or synthetic amino acids such as , β or α amino acids and cyclic derivatives. Incorporation of D- instead of L-amino acids into the polypeptide may increase its resistance to proteases. See, e.g., U.S. Patent 5,2 1 9,990.

The polypeptides and proteins of this invention may also be modified by various changes such as insertions, deletions and substitutions, either conservative or nonconservative where such changes might provide for certain advantages in their use.

In other embodiments, derivatives with amino acid substitutions which are less conservative may also result in desired derivatives, *e.g.*, by causing changes in charge, conformation and other biological properties. Such substitutions would include, for example, substitution of hydrophilic residue for a hydrophobic residue, substitution of a cysteine or proline for another residue, substitution of a residue having a small side chain for a residue having a bulky side chain or substitution of a residue having a net positive charge for a residue having a net negative charge. When the result of a given substitution cannot be predicted with certainty, the derivatives may be readily assayed according to the methods disclosed herein to determine the presence or absence of the desired characteristics.

Derivatives within the scope of the invention include proteins and peptides with amino acid sequences having at least eighty percent homology with the polypeptides and proteins described herein. More preferably the sequence homology is at least ninety percent, and still more preferably at least ninety-five percent.

Just as it is possible to replace substituents of the scaffold, it is also possible to substitute functional groups which decorate the scaffold with groups characterized by similar features. These substitutions will initially be conservative, *i.e.*, the replacement group will have approximately the same size, shape, hydrophobicity and charge as the original group. Non-sequence modifications may include, for example, *in vivo* or *in vitro* chemical derivatization of portions of naturally occurring polypeptides or proteins, as well as changes in acetylation, methylation, phosphorylation, carboxylation or glycosylation.

In a further embodiment the protein is modified by chemical modifications in which activity is preserved. For example, the proteins may be amidated, sulfated, singly or multiply halogenated, alkylated, carboxylated, or phosphorylated. The protein may also be singly or multiply acylated, such as with an acetyl group, with a farnesyl moiety, or with a fatty acid, which may be saturated, monounsaturated or polyunsaturated. The fatty acid may also be singly or multiply fluorinated. The invention also includes methionine analogs of the protein, for example the methionine sulfone and methionine sulfoxide analogs. The invention also includes salts of the proteins, such as ammonium salts, including alkyl or aryl ammonium salts, sulfate, hydrogen sulfate, phosphate, hydrogen phosphate, dihydrogen phosphate, thiosulfate, carbonate, bicarbonate, benzoate, sulfonate, thiosulfonate, mesylate, ethyl sulfonate and benzensulfonate salts.

Derivatives of the polypeptides and proteins may also include peptidomimetics. Such compounds are well known to those of skill in the art and include, for example, compounds produced through the substitution of certain R groups or amino acids in the protein with non-physiological, non-natural replacements. Such substitutions may increase the stability of such compound beyond that of the naturally occurring compound.

### Recombination

Recombinant expression vectors containing a nucleic acid sequence encoding polypeptide or protein can be prepared using well known methods. The expression vectors include a DNA sequence operably linked to suitable transcriptional or translational regulatory nucleotide sequences, such as those derived from a mammalian, microbial, viral, or insect gene. Examples of regulatory sequences include transcriptional promoters, operators, or enhancers, an mRNA ribosomal binding site, and appropriate sequences which control transcription and translation initiation and termination. Nucleotide sequences are "operably linked" when the regulatory sequence functionally relates to the DNA sequence encoding the polypeptide or protein of interest. For example, a promoter nucleotide sequence is operably linked to a DNA sequence encoding the protein or polypeptide of interest if the promoter nucleotide sequence controls the transcription of the DNA sequence encoding the protein of interest. The ability to replicate in the desired host cells, usually conferred by an origin of replication, and a selection gene by which transformants are identified, may additionally be incorporated into the expression vector.

In addition, sequences encoding appropriate signal peptides that are not naturally associated with the polypeptide or protein can be incorporated into expression vectors. For example, a DNA sequence for a signal peptide (secretory leader) may be fused in-frame to the DNA sequence of interest so that protein of interest is initially translated as a fusion protein comprising the signal peptide. A signal peptide that is functional in the intended host cells enhances extracellular secretion of the polypeptide or protein of interest. The signal peptide may be cleaved from the polypeptide or protein upon secretion of the polypeptide or protein from the cell.

Suitable host cells for expression include prokaryotes, yeast or higher eukaryotic cells. Appropriate cloning and expression vectors for use with bacterial, fungal, yeast, and mammalian cellular hosts are described, for example, in Pouwels *et al.* Cloning Vectors: A Laboratory Manual, Elsevier, New York, (1985). Cell-free translation system could also be employed to produce polypeptides or proteins using RNAs derived from DNA constructs disclosed herein.

Prokaryotes (also prokaryotes) include gram negative or gram positive organisms, for example, *E. Coli* or *Bacilli*. Suitable prokaryotic host cells for transformation include, for example, *E. coli, Bacillus subtilis, Salmonella typhimurium*, and various other species within the genera *Pseudomonas, Streptomyces*, and *Staphylococcus*. In a prokaryotic host cell, such as *E. coli,* a polypeptide or protein may include an N-terminal methionine residue to facilitate expression of the recombinant polypeptide in the prokaryotic host cell. The N-terminal Met may be cleaved from the expressed polypeptide or protein.

Expression vectors for use in prokaryotic host cells generally comprise one or more phenotypically selectable marker genes. A phenotypically selectable marker gene is, for example, a gene encoding a protein that confers antibiotic resistance or that supplies an autotrophic requirement. Examples of useful expression vectors for prokaryotic host cells include those derived from commercially available plasmids such as the cloning vector pBR322 (ATCC 37017). pBR322 contains genes for ampicillin and tetracycline resistance and thus provides simple means for identifying transformed cells. To construct an expression vector using pBR322, an appropriate promoter and a DNA sequence encoding a polypeptide or protein are-inserted into the pBR322 vector. Other commercially available vectors include, for example, pKK223-3 (Pharmacia Fine Chemicals, Uppsala, Sweden) and pGEMl (Promega Biotec, Madison, Wis., USA).

Promoter sequences commonly used for recombinant prokaryotic host cell expression vectors include 13-lactamase (penicillinase), lactose promoter system (Chang *et al.*, Nature 275:615, 1978; and Goeddel *et al.*, Nature 281:544, 1979), tryptopban (trp) promoter system (Goeddel *et al.*, Nucl. Acids Res. 8:4057, 1980; and EP-A-36776) and tac promoter (Maniatis, Molecular Cloning: A Laboratory Manual, Cold Spring Harbor Laboratory, p. 412, 1982). A particularly useful prokaryotic host cell expression system employs a phage LPL promoter and a cI857ts thermolabile repressor sequence. Plasmid vectors available from the American Type Culture Collection which incorporate derivatives of the LPL promoter include plasmid pHUB2 (resident in *E. coli* strain JMB9 (ATCC 37092)) and pPLc28 (resident in *E. coli* RR1 (ATCC 53082)). See Example 6 below in the Exemplification of the Invention for a discussion of a cassette-like plasmid system.

Polypeptides and proteins of the present invention alternatively may be expressed in yeast host cells, preferably from the *Saccharomyces* genus *(e.g., S. cerevisiae)*. Other genera of yeast, such as *Pichia, K lactis* or *Klayreromyces*, may also be employed. Yeast vectors will often contain an origin of replication sequence from a 2µ yeast plasmid, an autonomously replicating sequence (ARS), a promoter region, sequences for polyadenylation, sequences for transcription termination, and a selectable marker gene. Suitable promoter sequences for yeast vectors include, among others, promoters for metallothionein, 3-phosphoglycerate kinase (Hitzeman *et al.*, J. Biol. Chem. 255:2073, 1980) or other glycolytic enzymes (Hess *et al.*, J. Adv. Enzyme Reg. 7: 149, 1968; and Holland *et al.*, Biochem. 17 4900, 1978), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other suitable vectors and promoters for use in yeast expression are further described in Hitzeman, EPA-73,657 or in Fleer *et. al.*, Gene, 107:285-195 (1991); and van den Berg *et. al.*, Bio/Technology, 8:135-139 (1990). Another alternative is the glucose-repressible ADH2 promoter described by Russell *et al*. (J. Biol. Chem. 258:2674, 1982) and Beier *et al*. (Nature 300:724, 1982). Shuttle vectors replicable in both yeast and *E. coli* may be constructed by inserting DNA sequences from pBR322 for selection and replication in *E. coli* (Amp r gene and origin of replication) into the above described yeast vectors.

The yeast α-factor leader sequence may be employed to direct secretion of a polypeptide or protein. The a-factor leader sequence is often inserted between the promoter sequence and the structural gene sequence. See, *e.g.*, Kurjan *et al.*, Cell 30:933, 1982; Bitter *et al.*, Proc. Natl. Acad. Sci. USA 81 :5330, 1984; U.S. Pat. No. 204,546,082; and EP 324,274. Other leader sequences suitable for facilitating secretion of recombinant polypeptides from yeast hosts are known to those of skill in the art. A leader sequence may be modified near its 3' end to contain one or more restriction sites. This will facilitate fusion of the leader sequence to the structural gene.

Yeast transformation protocols are known to those of skill in the art. One such protocol is described by Hinnen *et al.*, Proc. Natl. Acad. Sci. USA 75: 1929, 1978. The Hinnen *et al*. protocol selects for Trp+ transformants in a selective medium, wherein the selective medium consists of 0.67% yeast nitrogen base, 0.5% casamino acids, 2% glucose, 10 µg/ml adenine and 20 µg/ml uracil.

Yeast host cells transformed by vectors containing ADH2 promoter sequence may be grown for inducing expression in a "rich" medium. An example of a rich medium is one consisting of 1 % yeast extract, 2% peptone, and 1% glucose supplemented with 80 µg/ml adenine and 80 µg/ml uracil. Derepression of the ADH2 promoter occurs when glucose is exhausted from the medium.

Mammalian or insect host cell culture systems can also be employed to express polypeptides or proteins of the present invention. Baculovirus systems for production of heterologous proteins in insect cells are reviewed by Luckow and Summers, Bio/Technology 6:47 (1988). Established cell lines of mammalian origin also may be employed. Examples of suitable mammalian host cell lines include the COS-7 line of monkey kidney cells (ATCC CRL 1651) (Gluzman *et al.*, Cell 23: 175, 1981), L cells, C127 cells, 3T3 cells (ATCC CCL 163), Chinese hamster ovary (CHO) cells, HeLa cells, and BHK (ATCC CRL 10) cell lines, and the CV-1/EBNA-I cell line derived from the African green monkey kidney cell line CVI (ATCC CCL 70) as described by McMahan *et al*. (EMBO J. 10: 2821, 1991).

Transcriptional and translational control sequences for mammalian host cell expression vectors may be excised from viral genomes. Commonly used promoter sequences and enhancer sequences are derived from Polyoma virus, Adenovirus 2, Simian Virus 40 (SV40), and human cytomegalovirus. DNA sequences derived from the SV40 viral genome, for example, SV40 origin, early and late promoter, enhancer, splice, and polyadenylation sites may be used to provide other genetic elements for expression of a structural gene sequence in a mammalian host cell. Viral early and late promoters are particularly useful because both are easily obtained from a viral genome as a fragment which may also contain a viral origin of replication (Fiers *et al.*, Nature 273:113, 1978). Smaller or larger SV40 fragments may also be used, provided the approximately 250 bp sequence extending from the Hind III site toward the Bgl 1 site located in the SV40 viral origin of replication site is included.

Exemplary expression vectors for use in mammalian host cells can be constructed as disclosed by Okayama and Berg (Mol. Cell. Biol. 3:28O, 1983). A useful system for stable high level expression of mammalian cDNAs in C 127 murine mammary epithelial cells can be constructed substantially as described by Cosman *et al.* (Mol. Immunol. 23:935, 1986). A useful high expression vector, PMLSV Nl/N4, described by Cosman *et al.*, Nature 312:768, 1984 has been deposited as ATCC 39890. Additional useful mammalian expression vectors are described in EP-A-0367566. The vectors may be derived from retroviruses. In place of the native signal sequence, a heterologous signal sequence may be added, such as the signal sequence for IL-7 described in U.S. Pat. No. 4,965,195; the signal sequence for IL-2 receptor described in Cosman *et al.*, Nature 312:768 (1984); the IL-4 signal peptide described in EP 367,566; the type I IL-1 receptor signal peptide described in U.S. Pat. No. 4,968,607; and the type II IL-1 receptor signal peptide described in EP 460,846.

### Purification and Isolation Methods

An isolated and purified polypeptide or protein according to the present invention can be produced by recombinant expression systems as described above or purified from naturally occurring cells. The polypeptides or proteins can be substantially purified, as indicated by a single protein band upon analysis by SDS-polyacrylamide gel electrophoresis (SDS-PAGE). One process for producing the polypeptides or proteins of the present invention comprises culturing a host celltransformed with an expression vector comprising a DNA sequence that encodes the polypeptide or protein under conditions sufficient to promote expression of the polypeptide or protein. The polypeptide or protein is then recovered from culture medium or cell extracts, depending upon the expression system employed. As is known to the skilled artisan, procedures for purifying a recombinant protein will vary according to such factors as the type of host cells employed and whether or not the recombinant protein is secreted into the culture medium. For example, when expression systems that secrete the recombinant protein are employed, the culture medium first may be concentrated using a commercially available protein concentration filter, for example, an Amicon or Millipore Pellicon ultrafiltration unit. Following the concentration step, the concentrate can be applied to a purification matrix such as a gel filtration medium.

Alternatively, an anion exchange resin can be employed, for example, a matrix or substrate having pendant diethylaminoethyl (DEAE) groups. The matrices can be acrylamide, agarose, dextran, cellulose or other types commonly employed in protein purification. Alternatively, a cation exchange step can be employed. Suitable cation exchangers include various insoluble matrices comprising sulfopropyl or carboxymethyl groups. Sulfopropyl groups are preferred. Finally, one or more reversed-phase high performance liquid chromatography (RP-HPLC) steps employing hydrophobic RP-HPLC media, (e.g., silica gel having pendant methyl or other aliphatic groups) can be employed to further purify the polypeptide or protein. Some or all of the foregoing purification steps, in various combinations, are well known and can be employed to provide an isolated and purified recombinant protein.

It is possible to utilize an affinity column comprising a binding protein specific to a polypeptide or protein of the invention to affinity-purify expressed polypeptides or proteins. Polypeptides or proteins can be removed from an affinity column using conventional techniques, e.g., in a high salt elution buffer and then dialyzed into a lower salt buffer for use or by changing pH or other components depending on the affinity matrix utilized.

Recombinant protein produced in bacterial culture is usually isolated by initial disruption of the host cells, centrifugation, extraction from cell pellets if an insoluble polypeptide, or from the supernatant fluid if a soluble polypeptide, followed by one or more concentration, salting-out,-ion exchange, affinity purification or size exclusion chromatography steps. Finally, RP-HPLC can be employed for final purification steps. Microbial cells can be disrupted by any convenient method, including freeze-thaw cycling, sonication, mechanical disruption, or use of cell lysing agents.

Transformed yeast host cells can be employed to express a polypeptide or protein as a secreted polypeptide in order to simplify purification. Secreted recombinant polypeptide from a yeast host cell fermentation can be purified by methods analogous to those disclosed by Urdal *et al*. (J. Chromatog. 296: 171, 1984). Urdal *et al*. describe two sequential, reversed-phase HPLC steps for purification of recombinant human IL-2 on a preparative HPLC column.

### Nucleic Acids

Antisense or sense oligonucleotides comprising a single-stranded nucleic acid sequence (either RNA or DNA) capable of binding to a target mRNA sequence encoding a polypeptide or protein of interest (forming a duplex) or to the sequence in the double-stranded DNA helix (forming a triple helix) can be made according to the invention. Antisense or sense oligonucleotides, according to the present invention, comprise a fragment of the polypeptide or protein coding region of the cDNA. Such a fragment generally comprises at least about 14 nucleotides, preferably from about 14 to about 30 nucleotides. The ability to create an antisense or a sense oligonucleotide, based upon a cDNA sequence for a given protein is described in, for example, Stein and Cohen, Cancer Res. 48:2659, 1988 and van der Krol *et al.*, BioTechniques 6:958, 1988.

Binding of antisense or sense oligonucleotides to target nucleic acid sequences results in-the formation of complexes that block translation (RNA) or transcription (DNA) by one of several means, including enhanced degradation of the duplexes, premature termination of transcription or translation, or by other means. The antisense oligonucleotides thus may be used to block expression of the polypeptides or proteins of the invention. Antisense or sense oligonucleotides further comprise oligonucleotides having modified sugar-phosphodiester backbones (or other sugar linkages, such as those described in WO91/06629) and wherein such sugar linkages are resistant to endogenous nucleases. Such oligonucleotides with resistant sugar linkages are stable *in vivo* (*i. e.*, capable of resisting enzymatic degradation) but retain sequence specificity to be able to bind to target nucleotide sequences. Other examples of sense or antisense oligonucleotides include those oligonucleotides which are covalently linked to organic moieties, such as those described in WO 90/10448, and other moieties that increases affinity of the oligonucleotide for a target nucleic acid sequence, such as poly-(L-lysine). Further still, intercalating agents, such as ellipticine, and alkylating agents or metal complexes may be attached to sense or antisense oligonucleotides to modify binding specificities of the antisense or sense oligonucleotide for the target nucleotide sequence.

Antisense or sense oligonucleotides may be introduced into a cell containing the target nucleic acid sequence by any gene transfer method, including, for example, CaPO₄ -mediated DNA transfection, electroporation, or by using gene transfer vectors such as Epstein-Barr virus. Antisense or sense oligonucleotides are preferably introduced into a cell containing the target nucleic acid sequence by insertion of the antisense or sense oligonucleotide into a suitable retroviral vector, then contacting the cell with the retrovirus vector containing the inserted sequence, either *in vivo* or ex vivo. Suitable retroviral vectors include, but are not limited to, the murine retrovirus M-MuLV, N2 (a retrovirus derived from M-MuLV), or the double copy vectors designated DCTSA, DCT5B and DCT5C.

Sense or antisense oligonucleotides also may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell.

Alternatively, a sense or an antisense oligonucleotide may be introduced into a cell containing the target nucleic acid sequence by formation of an oligonucleotide-lipid complex, as described in WO 90/10448. The sense or antisense oligonucleotide-lipid complex is preferably dissociated within the cell by an endogenous lipase.

The present invention includes polynucleotides capable of hybridizing under stringent conditions, preferably highly stringent conditions, to the polynucleotides described herein. Highly stringent conditions include, for example, 0.2xSSC at 65°C; stringent conditions include, for example, 4xSSC at 65°C or 50% formamide and 4xSSC at 42°C. Preferably, such hybridizing nucleotides are at least 70% identical (more preferably at least 80% identical; still more preferably 85%, 90% or 95% identical) with the polynucleotide of the invention to which they hybridize.

The invention also includes allelic variants of the disclosed polynucleotides or proteins; that is-naturally occurring and non-naturally occurring alternative forms of the isolated polynucleotide which also encode proteins which are identical, homologous or related to that encoded by the polynucleotides of the invention.

### Homology or Identity

To determine the percent identity of two amino acid sequences or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, 90% or 95% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch *(J. Mol. Biol*. (48):444-453 (1970)) algorithm which has been incorporated into the GAP program in the GCG software package (available at http://www.gcg.com), using either a Blossom 62 matrix or a PAM250 matrix, and a gap weight of 16, 14, 12, 10, 8, 6, or 4 and a length weight of 1, 2. 3, 4. 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. In another embodiment, the percent identity between two amino acid or nucleotide sequences is determined using the algorithm of E. Meyers and W. Miller (CABIOS, 4:11-17 (1989) which has been incorporated into the ALIGN program (version 2.0) (available at http://vega.igh.cnrs.fr/bin/align-guess.cgi), using a PAM120 weight residue table, a gap length penalty of 12 and a gap penalty of 4.

The nucleic acid and protein sequences of the present invention can further be used as a "query sequence" to perform a search against public databases to, for example, identify other family members or related sequences. Such searches can be performed using the NBLAST and XBLAST programs (version 2.0) of Altschul, *et al.* (1990) *J. Mol. Biol*. 215 :403-10. BLAST nucleotide searches can be performed with the NBLAST program, score = 100, wordlength = 12 to obtain nucleotide sequences homologous to MSP-18 nucleic acid molecules of the invention. BLAST protein searches can be performed with the XBLAST program, score = 50, wordlength = 3 to obtain amino acid sequences homologous to MSP-18 protein molecules of the invention. To obtain gapped alignments for comparison purposes, Gapped BLAST can be utilized as described in Altschul *et al.*, ( 1997) *Nucleic Acids Res*. 25(17):3389-3402. When utilizing BLAST and Gapped BLAST programs, the default parameters of the respective programs (*e.g.*, XBLAST and NBLAST) can be used. See http://www.ncbi.nlm.nih.gov. Additionally, the "Clustal" method (Higgins and Sharp, Gene, 73:237-44, 1988) and "Megalign" program (Clewley and Arnold, Methods Mol. Biol, 70: 119-29, 1997) can be used to align sequences and determine similarity, identity, or homology. See Example 10 below in the Exemplification of Invention.

### Gene Therapy

The nucleic acid molecules of the invention can be inserted into vectors and used as gene therapy vectors. Gene therapy vectors can be delivered to a subject by, for example, intravenous injection, local administration (see U.S. Patent 5,328,470) or by stereotactic injection (see *e.g.*, Chen *et al.* (1994) Proc. Natl. Acad. Sci. USA 91 :3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g.*, retroviral vectors, the pharmaceutical preparation can include one or more cells which produce the gene delivery system. The pharmaceutical compositions can be included in a container, pack, or dispenser together with instructions for administration.

The present invention discloses a method for regulating the conformation of a target protein by contacting the target protein with a molecular chaperone system having a first protein of at least one Hsp100 protein or a homolog thereof and a second protein of at least one Hsp70 protein or a homolog thereof to regulate the conformation of the target protein. In a preferred embodiment, the first protein comprises a prokaryotic homolog of the Hsp100 protein, and more preferred, the homolog is a Clp homolog. The Clp homolog is preferably ClpA, ClpX, or more preferably, ClpB. In a preferred embodiment, the second protein is a prokaryotic homolog of the Hsp70 protein. More preferably, the prokaryotic homolog is DnaK. In an additional preferred embodiment, the molecular chaperone system further has at least one cochaperone. Preferred cochaperones are DnaJ, GrpE or ClpS and more preferably DnaJ and GrpE. Cochaperones that are particularly preferred are DnaJ and GrpE.

In an additional embodiment, the target protein is recombinantly expressed in a host cell, and the target protein can be aggregated due to over expression in the host cell. In a further preferred embodiment, the first (Hsp100 protein) and second (Hsp70 protein) proteins are recombinantly expressed in the host cell. The host cell is preferably an *E. Coli, S. Cerevisiae, B. Subtilis*, plant, insect, yeast, or mammalian cell. More preferably, the host cell is *E. Coli.*

The invention provides a system of recombinant expression vectors. In accordance with one embodiment, a single recombinant expression vector encodes at least one Hsp100 protein or homologue thereof and at least one Hsp70 protein or homologue thereof. The expression vector can further encode one or more chaperones for one or both of the Hsp100 and Hsp70 proteins, as well as protein of interest. In another embodiment, a single vector is used to express each protein component of the molecular chaperone system. In another embodiment, the vector can express one, two, three, *etc.* Hsp100 proteins or, likewise, one, two, three, *etc.* Hsp70 proteins, and all combinations of chaperone and cochaperone proteins in between. A host cell can be transformed with one or more vectors of the system. In a preferred embodiment, a cassette-like plasmid system developed by Bujard and coworkers (Lutz and Bujard, 1997) is used to deploy the recombinant vector system. More preferably, the vector system is further capable of recombinantly expressing the GroEL-GroES system or a homolog thereof, an individual protein thereof, a homolog thereof or Trigger Factor or a homolog thereof. A discussion of the GroEL-GroES system can be found in Bukau and Horwich, Cell (1998).

In the present invention, the target protein can be aggregated or misfolded. The target protein can become aggregated due to stress conditions, such as heat stress, oxidative stress, overexpression, mutation or disease. In a preferred embodiment, the target protein is disaggregated *in vitro*.

The present invention further provides a vector system encoding a molecular chaperone system comprising at least one Hsp100 protein or a homolog thereof and at least one Hsp70 protein or a homolog thereof. The vector system can contain an inducible promoter so that expression can be controlled by the presence or absence of a stimulus.

The vector system can be used to transform a cell. In a preferred embodiment, the cell is protease deficient. More preferably, the protease deficiency is caused by a mutation in the *rpoH* gene, and more preferably, the mutation is a chromosomal deletion in the *rpoH* gene in a cell that additionally has an IS-element in the promoter region of the GroEL-GroES operon, thereby permitting cell growth.

In one embodiment of the invention, the host cell can recombinantly express a protein of interest. The protein of interest is likely to misfold or aggregate in the absence of recombinant expression of the molecular chaperone system. In another embodiment, the expressed molecular chaperone system inhibits aggregation or misfolding of the protein of interest. In a preferred embodiment, the aggregation and misfolding of the protein of interest is inhibited to increase the yield of the active conformation of the protein of interest compared to a cell where the molecular chaperone system is not recombinantly expressed. In another embodiment, the molecular chaperone system disaggregates and refolds the aggregated protein. In a preferred embodiment, the protein of interest is Malate Dehydrogenase. In accordance with the invention, the cell can be any prokaryotic or eukaryotic cell, and more preferably the cell is an *E Coli, S. Cerevisiae, B. Subtilis*, plant, insect, yeast, or mammalian cell.

The present invention further provides a method for disaggregating an aggregated protein *in vitro* by contacting the aggregated protein with a molecular chaperone system having at least one Hsp100 protein or a homolog thereof and at least one Hsp70 protein or a homolog thereof to disaggregate the aggregated protein. In a preferred embodiment, the method further folds the disaggregated protein into an active form.

In another embodiment, the invention provides a method for increasing the solubility of a recombinantly expressed protein by coexpressing a molecular chaperone system having at least one Hsp100 protein or a homolog thereof and at least one Hsp70 protein or a homolog thereof to increase the solubility of the recombinantly expressed protein.

In still another embodiment, the invention is a method for preventing cell death caused by misfolding and aggregation of a protein still by administering a therapeutically effective amount of a molecular chaperone system having at least one Hsp100 protein or a homolog thereof and atleast one Hsp70 protein or a homolog thereof to prevent the misfolding and aggregating of a protein.

The invention also provides a method of treating a disease in an animal caused by misfolding and aggregation of a protein by administering a therapeutically effective dose of a molecular chaperone system having at least one Hsp100 protein or a homolog thereof and at least one Hsp70 protein or a homolog thereof to thereby treat the disease. In a preferred embodiment, the animal is a mammal such as a human, a rodent, a cat, or a dog. Diseases that can be treated in accordance with the invention include Creutzfeld-Jacob's disease, Alzheimer's disease, Huntington's disease, Ataxia type-1, cystic fibrosis and cancer. The therapeutically effective dose is preferably delivered with a pharmaceutically acceptable carrier. More preferably, the pharmaceutically acceptable carrier is capable of targeting a misfolded protein. Additionally, the present invention provides methods of gene therapy and compositions useful to carryout gene therapy by a polynucleic acid molecule encoding a molecular chaperone system as hereinabove described.

A further embodiment of the invention is a pharmaceutical composition having an effective dose of a molecular chaperone system comprising at least one Hsp100 protein or a homolog thereof and at least one Hsp70 protein or a homolog thereof and a pharmaceutically acceptable carrier.

Another embodiment of the invention is a method for increasing the folding efficiency of a protein *in vitro* by adding Trigger Factor with or without at least one Hsp70 protein or homolog thereof, to an *in vitro* protein synthesis system to increase the folding efficiency of proteins *in vitro*. In a preferred embodiment, in addition to Trigger Factor, a molecular chaperone system comprising at least one Hsp70 protein or a homolog thereof is added to the *in vitro* synthesis system. The folding efficiency of a protein in a cell can be increased by recombinantly expressing Trigger Factor with or without at least one Hsp70 protein or homolog thereof, in the cell to thereby increase the folding efficiency of the protein in the cell. The cell can be prokaryotic or eukaryotic. In a preferred embodiment, in addition to Trigger Factor, a molecular chaperone system comprising at least one Hsp70 protein or a homolog thereof is recombinantly expressed.

The invention provides polynucleic acid molecules related to the polynucleotide sequence of SEQ. ID. NO. 1. In an embodiment, the invention provides an isolated polynucleic acid molecule selected from the group consisting of (a) a polynucleotide comprising the nucleotide sequence of SEQ ID NO: 1; (b) a polynucleotide that is antisense to the polynucleotide of (a); (c) a polynucleotide having 80% identity to the polynucleotide of (a) or (b); (d) a polynucleotide encoding the protein of SEQ ID NO:2; (e) a polynucleotide encoding a polypeptide having 80% identity to the protein of SEQ ID NO:2; (f) a polynucleotide having 80% identity to the nucleic acid of (a)-(e) encoding a protein capable of regulating the activity of Hsp100 protein or a homolog thereof in disaggregation; and (g) a polynucleotide that hybridizes under stringent conditions to any one of the polynucleotides specified as (a)-(f). In a preferred embodiment, the polynucleotides of the invention are at least 70% identical; more preferably at least 80% identical, still more preferably 85%, 90%, or 95% identical to the amino acid sequence of SEQ ID NO: 1.

The invention further provides polypeptides and proteins related to SEQ. ID. NO.2. In an embodiment, the invention provides an isolated polypeptide selected from the group consisting of: (a) a polypeptide comprising the amino acid sequence of SEQ ID NO:2; (b) a polypeptide having 80% identity to the amino acid sequence of SEQ ID NO:2; (c) a polypeptide variant of the amino acid sequence of SEQ ID NO:2; (d) a polypeptide variant of the amino acid sequence of SEQ ID NO:2 capable of regulating the activity of a Hsp100 protein or a homolog thereof in disaggregation; (e) a polypeptide variant of the amino acid sequence of SEQ ID NO:2 capable of co-chaperoning ClpA; (0 a polypeptide encoded by the polynucleotide SEQ ID NO:1; (g) a polypeptide encoded by a polynucleotide having 80% identity to the sequence of SEQ ID NO: 1; and (h) a polypeptide encoded by a polynucleotide which hybridizes under stringent conditions to polynucleotide having the sequence of SEQ ID NO: 1. In an embodiment, the variant or derivative is an insertion, a deletion, or a substitution variant or derivative. In a preferred embodiment, the polypeptides of the invention are at least 70% identical; more preferably at least 80% identical; still more preferably 85%, 90%, or 95% identical to the amino acid sequence of SEQ ID NO: 2.

Additionally, a molecular chaperone system is provided to regulate protein conformation having a first protein of at least one Hsp100 protein or a homolog thereof and a second protein of at least one Hsp70 protein or a homolog thereof.

An additional embodiment is the use of ClpS or homologs or derivatives thereof to regulate the activity of a Hsp100 protein. In a preferred embodiment the Hsp100 protein is ClpA, ClpB or ClpX. In a more preferred embodiment, the Hsp100 protein is ClpA.

An additional embodiment is a method for increasing the folding efficiency of a protein in *E. coli* cells as well as in in vitro protein synthesis systems with mutationally alterated content of cytosolic chaperones (e.g. in mutants lacking the regulator gene, rpoH).

In this regard the trigger factor (TF) in combination with the DnaK (Hsp70 protein) chaperone system (consisting of DnaK, DnaJ, GrpE) is important for protein folding; cellular substrates for these two chaperone systems were identified.

Moreover, ClpB (Hsp 100 protein) in combination with the DnaK system is important for protein folding and refolding at 30°C after aggregation has occurred. The DnaK system together with ClpB are furthermore suitable to increase the survival of E. *coli* at severe heat shock conditions.

Further, the DnaK system and the GroEL system do not act primarily in sequential fashion in assisting the folding of newly synthesized proteins but rather assist the folding of significantly distinct substrate populations.

Another result of the present invention is that Lon and ClpXP are the major cytosolic proteases capable to eliminate the aggregated proteins accumulating in chaperone gene mutants.

For the experiments carried out to prove the findings two strains were used. First, a *tig::kan* strain lacking TF which in addition has the *dnaK dnaJ* operon replaced by an artificial operon allowing control of expression by the inducer IPTG (Deuerling *et al.*, 1999). Second, a *rpoH* strain lacking the heat shock transcription factor, sigma 32, that is responsible for expression of all major cytosolic chaperones and proteases except TF. This published strain (Kusukawa and Yura, 1988) is viable at 30°C and 37°C because of a suppressor mutation allowing constitutive expression of GroEL at high levels. The findings using these strains and their newly constructed derivatives are as follows.

About 10% of total cytosolic proteins are found in the insoluble pellet in *tig::kan* cells (lacking TF) depleted from DnaK at 37°C. The amount of aggregated protein was lower at 30°C (1.2%) indicating that the requirement for DnaK/TF is temperature dependent. These findings indicate that for efficient folding of proteins in *E. coli*, TF and the DnaK system become more important at higher growth temperature. They also suggest that the folding efficiency of recombinant proteins synthesized in *E. coli* based *in vivo* and *in vitro* systems may be improved by supplementation with TF and/or the DnaK system especially when the synthesis is performed at higher temperature.

Further, a large scale identification of the aggregated proteins identified at 37°C in *tig::kan* cells depleted from DnaK by 2D-gel electrophoresis and subsequent mass spectrometry (were performed). More than 92 proteins were identified which participate in a variety of basic cellular processes including transcription, translation and metabolism. A common striking feature is the size dependency of this protein class which could cover the entire molecular weight range, i.e. from a few Da to several hundred kDa. Especially high molecular weight proteins built up by multiple domains require the assistance of TF and/or DnaK to fold productively. For the E. *coli* proteins identified as well as for heterologous proteins, overproduction of TF and the DnaK system is recommended to increase the yield of native recombinant proteins under high level production conditions in biotechnological application. Examples for appropriate proteins, called aggregation-prone proteins, are Glutamate-synthase (m.w. ca. 167 kDa), RNA-polymerases (ca. 150 - 160 kDA), Thr-tRNA-synthetase (ca. 75 kDa).

There is a functional relationship between the DnaK system, the GroEL system (consisting of GroEL and GroES) and TF in assisting the folding of newly synthesized proteins in *E. coli* cells. This relationship was investigated by altering the cellular levels of chaperones individually or in combination and analyzing chaperone-substrate interactions by co-immunoprecipitation with chaperone-specific antibodies.

Previous published experiments already showed that TF and the DnaK system cooperate in protein folding (Deuerling *et al.*, 1999; Teter *et al.*, 1999). We now find that (i) deletion of the *dnaK* gene or (ii) depletion of DnaK and DnaJ in a strain with an IPTG regulatable *dnaK dnaJ* operon, was sufficient to increase protein aggregation as compared to wild type cells. These findings indicate that there is already a distinct contribution of the DnaK system to protein folding which cannot be compensated by presence of the other cellular chaperones. However, consistent with earlier findings (Deuerling *et al.*, 1999), a much stronger protein aggregation occurred in *tig* cells depleted for DnaK and DnaJ.

To analyze the functional relationship between DnaK and GroEL, the *rpoH* mutant strain containing the suppressor mutation allowing expression of GroEL was used. In this strain about 5% of total soluble cell protein aggregated at 30°C was found. IPTG regulated overproduction of GroEL and GroES from multicopy plasmids did not significantly decrease the amount of aggregated proteins. In contrast, the IPTG induced overproduction of the DnaK system was sufficient to efficiently suppress aggregation of almost all aggregation-prone proteins. The concomitant production of both, the DnaK system and ClpB, was even more efficient in suppressing aggregation of proteins at 30°C as well as after heat shock to 42°C and 50°C.

Further, *in vivo* substrate populations associated with GroEL and with DnaK in *rpoH* cells containing different levels of plasmid-encoded DnaK system were identified. This identification was performed by co-immunoprecipitation of radiolabeled cellular proteins with GroEL- or DnaK- specific antisera. The result is that the substrate populations of DnaK and GroEL differ significantly although an overlap in these populations is not excluded. Thus, in accordance with earlier findings (Deuerling *et al.*, 1999; Ewalt *et al.*, 1997) GroEL has an upper size limitation for substrates of about 65 kDa. In contrast, the proteins interacting with DnaK are enriched in proteins with high molecular weigth >60 kDa. Furthermore, the amount of proteins interacting transiently with GroEL was not affected by changing the levels of DnaK with or without TF present. The loading of GroEL with substrates thus does not require the presence of the DnaK system and TF. These findings indicate the existance of distinct folding pathways for newly synthesized proteins in *E. coli.* Some proteins interact with the DnaK system and perhaps ClpB (when aggregated), while other proteins interact with the GroEL system. Trigger factor as a ribosome-associated chaperone also plays an essential role in folding of both types of populations.

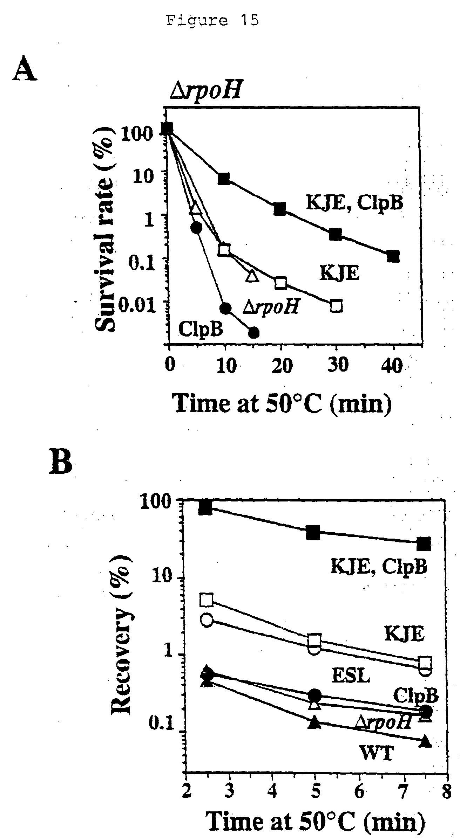

The role of chaperones in cell survival at heat stress conditions using the *ΔrpoH* strain mentioned before is of essential importance. This strain showed high thermosensitivity (Fig. 15). Expressing the DnaK system with ClpB was most effective in suppressing cell killing at heat shock temperatures (Fig. 15) and allowed the efficient prevention and reversion of aggregation of cellular proteins (Fig. 15) and reactivation of a thermolabile reporter enzyme (firefly luciferase; Fig. 15). The increased production of the DnaK system and ClpB and their homologs are useful to increase the stress resistance including survival at and recovery from thermal stress of not only the *ΔrpoH* strain but also of wild type *E. coli.* We expect that overproduction of homologs of the Dnak system and ClpB can also increase stress restistance of other prokaryotic and eukaryotic cells.

Another result of the present invention is that Lon and CIpXP are the major cytosolic proteases responsible for the elimination of aggregation-prone proteins which accumulate in *rpoH* mutant cells. Consequently it is useful to design *E. coli* strains or *E. coli* based *in vitro* protein synthesis systems for the production of recombinant proteins accordingly. In particular, ommission of these proteases will increase the yield of unstable and aggregation-prone recombinant proteins.

The following examples are provided for the purposes of illustration and are not intended to and should not be construed to limit the scope of the present invention or the claims which follow.

### Exemplification of the Invention

An investigation was conducted into the potential of cytosolic chaperones of *E. coli* to fold proteins to native structures, to prevent and reverse the aggregation of thermally denatured proteins, and to assist the refolding of these substrates. The analysis of thermal denaturation and aggregation of proteins is a well established experimental approach to study protein misfolding as it also occurs during stress treatment of cells. With respect to the role of chaperones, the temperature induced aggregation of proteins resembles the aggregation and inclusion body formation of recombinant proteins that frequently occurs upon overproduction in bacteria. Furthermore, it was observed that aggregates of thermally denatured proteins (e.g., Malate Dehydrogenase, MDH) show increased staining with Congo red, a widely used marker stain indicative for amyloid fibers. In particular, Congo red reacts with anti parallel 13-sheet structures which are implicated in fiber formation (Turnell and Finch, 1992). Aggregates of heat denatured proteins may thus have increased anti parallel 13-sheet contents and this alteration in secondary structure is shared with amyloid and prion forming proteins. Therefore analysis of heat denatured proteins is considered relevant for studying amyloid and prion metabolism.

For use in *E. coli*, a set of compatible plasmids was constructed which allowed the independently regulated expression of genes encoding recombinant proteins and genes encoding ClpB and the DnaK system. Also constructed were plasmids that encode additional chaperone genes, in particular the GroEL-GroES system and the Trigger Factor, which may be useful as well to increase the yield of soluble protein in vivo.

Additionally, an *E. coli* strain was constructed for high level production of unstable recombinant proteins. This strain carries the mentioned. plasmids and additionally a chromosomal deletion in the regulatory *rpoH* gene which renders this strain deficient in the activity of major cytosolic proteases in a cell that additionally has an IS-element in the promoter region of the GroEL-GroES operon, thereby permitting cell growth.

### Example 1: DnaK chaperone system is effective for preventing protein aggregation

This experiment assessed the ability of the DnaK chaperone system to prevent protein aggregation as compared to other chaperones/chaperone systems. The DnaK system comprises the heat shock protein DnaK (Hsp70), co-chaperone DnaJ (Hsp40), and co-chaperone GrpE. The GrpE co-chaperone regulates the ATPase activity of DnaK and thereby assists DnaK in the refolding of protein substrates (Bukau and Horwich, 1998).

The experimental approach followed published procedures (Hesterkamp and Bukau, EMBO J., 17, pp. 4818-4828) and relies on the identification of aggregated proteins by centrifugation. 35S-methionine labeled soluble extracts of *E. coli* wild type cells (4 mg/ml) were pre-incubated for 5 min. at 30°C in the absence or presence of cytosolic chaperones. ATP (10 mM) was added 2 min. prior to heat-shock. Samples were then shifted for 15 min. to 45°C. Soluble and insoluble protein material was separated by centrifugation. Aggregated proteins were pelleted by centrifugation for 15 min. at 15000 g and 4°C. Pellets were washed twice with ice-cold breakage buffer and analyzed afterwards by 1-D and 2-D gel electrophoresis.

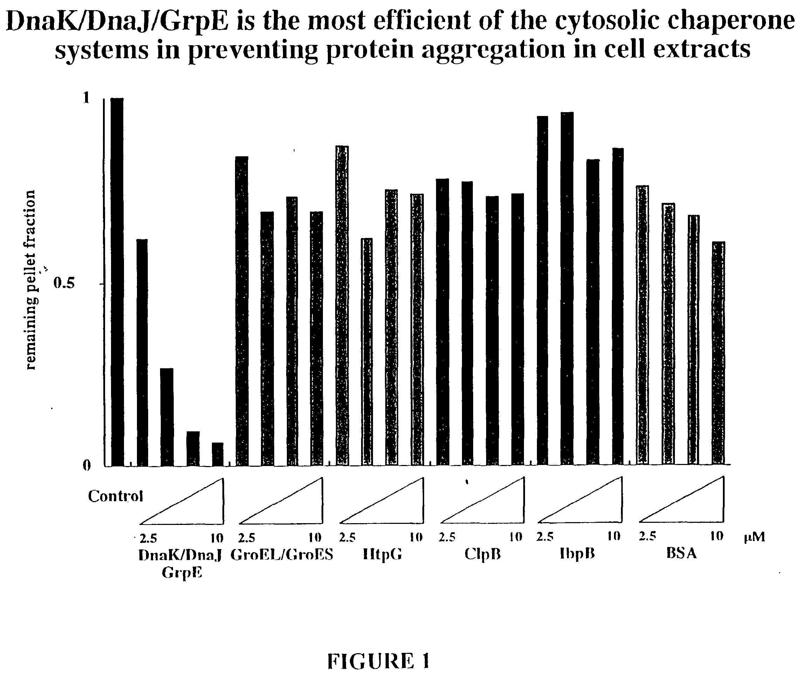

The amount of aggregated proteins was determined by scintillation counting. Figure 1 compares the ability of different chaperones to prevent protein aggregation in *E. coli* cell extracts. As shown in Figure 1, the DnaK system is an effective cytosolic chaperone system for preventing aggregation of thermally denatured *E. coli* proteins in cell extracts, and is more efficient than cytosolic chaperones GroEL/GroES, HtpG (Hsp90), ClpB, and IbpB in preventing aggregation under conditions of approximately equimolar concentrations of chaperones and heat-labile protein substrates in cell extracts.

The results of this experiment indicated the use of the DnaK system as an element in the technological approach to increase solubility of recombinant proteins.

### Example 2: Identification of thermosensitive proteins, the aggregation of which is prevented by the DnaK system

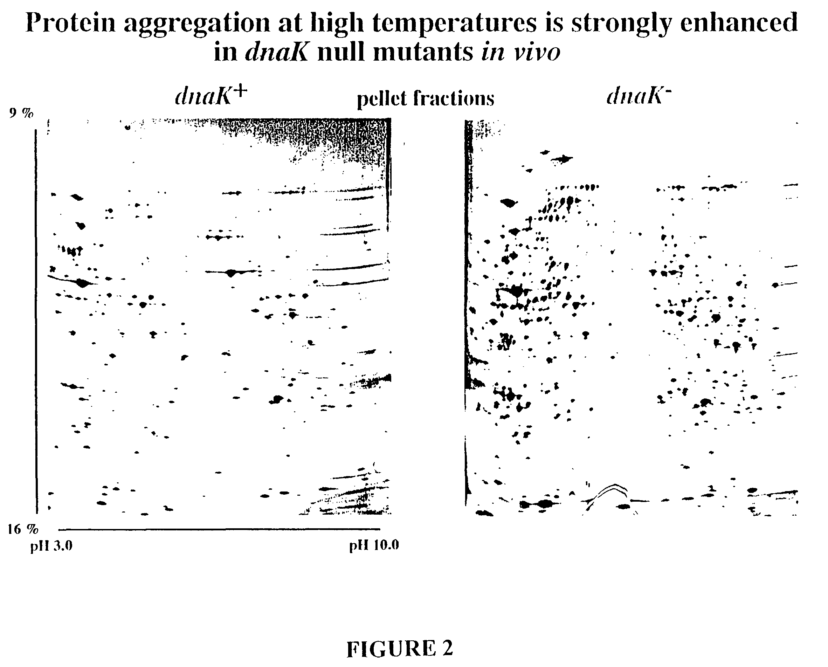

*In vivo* experiments were conducted to identify more than 60 thermosensitive *E. coli* proteins which were prevented by the DnaK system from temperature-induced aggregation. The proteins so identified are listed in Table 2. Protein identification was achieved by analysis of aggregated proteins accumulating in ΔdnaK mutants after shift to the non-permissive heat shock temperature (42°C). *E. coli* wild type and *dnaK* mutant cells were grown exponentially in LB medium at 30°C and heat-shocked to 42°C for 60 min. Insoluble cell fractions (including membrane proteins) were prepared and analyzed by two-dimensional gel electrophoresis following standard protocols. Figure 2 shows the aggregated proteins as Coomassie-stained spots after two-dimensional gel electrophoresis of pellet fractions of wild type and *dnaK*⁻ cells.

**Table 2**

| Identification of natural DnaK substrates in vivo | | | |
|---|---|---|---|
| Name | Size | [kDa] Function | Oligomeric State |
| RpoB | 151 | RNA-Polymerase | α2ββ |
| PutA | 145 | Proline-Dehydrogenase | |
| PurL | 142 | Formylglycinamidine-Synthase | Monomer |
| NarG | 141 | Nitrate-Reductase (Respiratory chain) | α-Subunit, Protein |
| MetH | 137 | Methionine biosynthesis | Monomer |
| NifJ | 130 | Oxidoreductase (Respiratory chain) | |
| MFD | 130 | Transcription repair coupling factor | |
| CarB | 118 | Carbamoylphosphate-Synthetase | Large Subunit of α4β4 |
| Odol | 106 | E1 componentofOxoglutarate-Dehydrogenase | Homodimer,Protein |
| Odp 1 | 100 | E I component of Pyruvate-Dehydrogenase | Homodimer, Protein |
| AdhE | 96 | Alcohol-Dehydrogenase | 40 Subunits |
| Aco2 | 94 | Aconitase | Monomer |
| Lon | 88 | ATP-dependent protease | Tetramer |
| MetE | 85 | Methionine biosynthesis | Monomer |
| YghF | 82 | Unknown | |
| EF-G | 78 | Elongation Factor of translation | Monomer |
| Pta | 77 | Acetyltransferase | |
| Syt | 75 | Threonine-tRNA-synthetase | Homodimer |
| Tktl | 72 | Transketolase I | |
| Dxs | 68 | Transketolase | |
| SfcA | 66 | Probable Malate-Oxidoreductase | |
| TypA | 66 | Unknown, EF-G Homologue | |
| Odp2 | 66 | E2 component of Pyruvate-Dehydrogenase | 24 Subunits, Protein |
| DhsA | 65 | Succinate-Dehydrogenase | Proein complex |
| SypA | 64 | Proline-tRNA-Synthetase | Homodimer |
| FumA | 61 | Fumarase | Homodimer |
| GuaA | 59 | Glutamine-Amidotransferase | Homodimer |
| Sykl | 57 | Transcriptional activator | Homodimer |
| NusA | 55 | Terminator and Antiterminator of transcription | Association with RNA- |
| AsnS | 53 | Asparagine-tRNA-Synthetase | Homodimer |
| GlnA | 52 | Glutamine-Synthetase | 12 Subunits |
| ImdH | 52 | IMP-Dehydrogenase | Tetramer |
| G 1788049 | 50 | Unknown | |
| HslU | 50 | Subunit of ATP-dependent Protease | Hexamer |
| ClpX | 47 | Subunit of ATP-dependent Protease | Hexamer |
| ThrC | 47 | Threonine biosynthesis | |
| DeoA | 47 | Thymidine-Phospho∼ylase | Homodimer |
| Rho | 47 | Tennination of transcription | Hexamer, Association |
| MurA | 45 | Cell wall synthesis | |
| SerA | 44 | 3-Phosphoglycerat-Dehydrogenase | Tetramer |
| Tgt | 43 | Quenine-tRNA-Ribosyl-Transferase | Homodimer/Homotrimer |
| Glf | 43 | LPS synthesis | |
| CarA | 42 | Carbamoylphosphate-Synthetase | Small Subunit of α4β4 |
| RfbB | 41 | DTDP-Glucose-4,6-Dehydratase | |
| Pgk | 41 | Phosphoglycerat-Kinase | Monomer |
| FtsZ | 40 | Cell division | Ring-like structure |
| RpoA | 37 | RNA-Polymerase | α2ββ' |
| AsnA | 37 | Asparagine-Synthetase | Homodimer |
| GAP-DH | 36 | 3-Glyceralaldehyd-Dehydrogenase | Tetramer |
| MhpE | 34 | 3-Hydroxyphenylpropionate-Degradation | |
| EntB | 33 | Isochorismatase | |
| MetF | 33 | Methionine biosynthesis | Tetramer |
| GatY | 31 | Fructose-bisphospate-Aldolase | |
| MinD | 30 | Cell Division | Ring-likestructure |
| SucD | 30 | Succinyl-CoA-Synthetase | α-Subunit of α2α2 |
| GpmA | 29 | Phosphoglycerate-Mutase | |
| DeoC | 28 | Deoxyribosephospate-Aldolase | |
| ArcA | 27 | Response Regulator (Oxygen control) | |
| UbiB | 26 | Flavoprotein-Oxidoreductase | Monomer. |
| PyrH | 26 | UMP-Kinase | Hexamer |
| NusG | 21 | Antitenninator of transcription | Assoc n w/RNA-Polymerase |
| IbpB | 16 | small HSP (chaperone) | |

Figure 2 demonstrates that protein aggregation at high temperatures is strongly enhanced in *dnaK* null mutants *in vivo*. These findings and the reported limited capacity of the DnaK system in wild type cells (Tomoyasu *et al.*, 1998), suggest that in biotechnological applications the yield of soluble conformers of these proteins after overproduction, *e.g*.,. in *E. coli*, will increase when the DnaK system is co-overproduced.

### Example 3: Determination of the relative abilities of various chaperones to disaggregate and refold aggregated proteins in vitro - Combined action of the DnaK system and ClpB

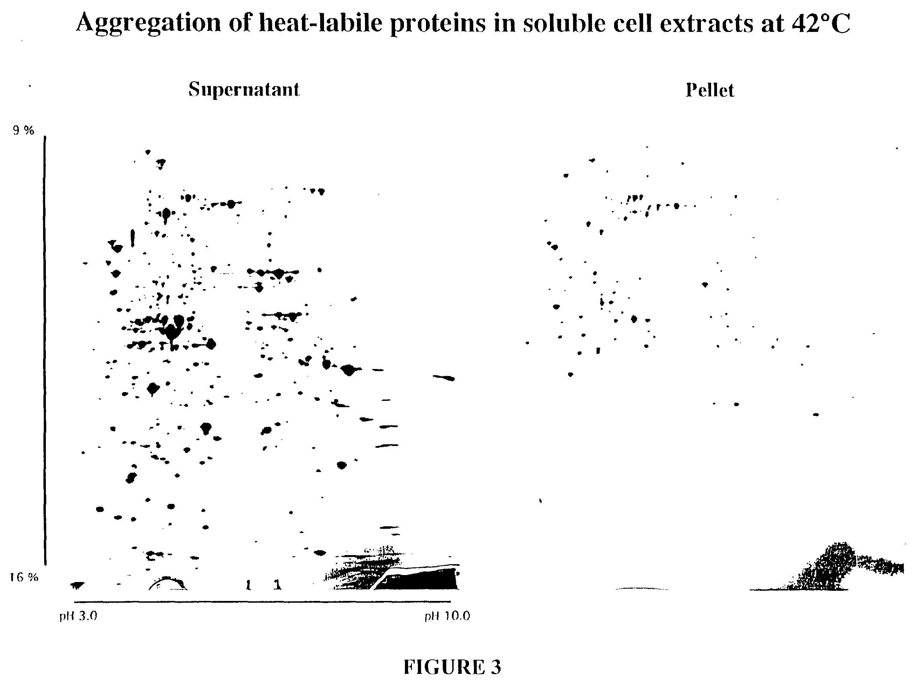

*In vitro* experiments were performed to determine the ability of various chaperones to disaggregate and refold aggregated proteins. In one set of experiments, total soluble extracts of *E. coli* wild type cells were preincubated at 30°C for 5 min. and heat-shocked to 45°C for 15 min. Soluble and insoluble fractions were separated by centrifugation and analyzed by two-dimensional gel electrophoresis following standard protocols. As shown in Figure 3, the heat shock treatment caused extensive aggregation of approximately 10 - 20 % of the proteins in the lysate, including more than 50 different protein species.

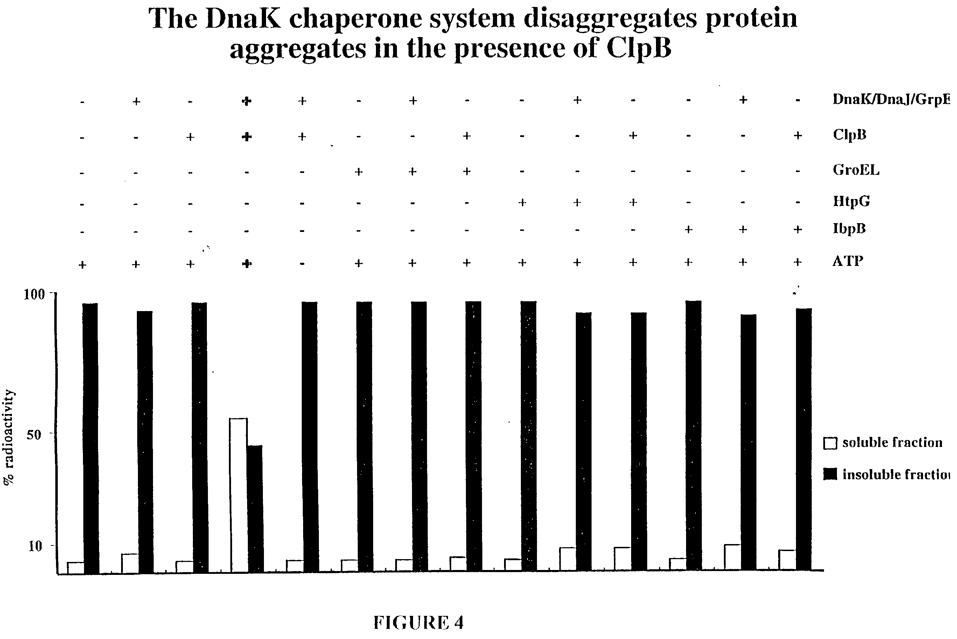

³⁵S-methionine labeled soluble extracts of *E. coli* wild type cells were preincubated at 30°C for 5 min. and heat-shocked to 45°C for 15 min. Aggregated proteins were isolated by centrifugation and resuspended in reaction buffer (50 mM HEPES pH 7.6; 150 mM KCI; 20 mM MgC12). The DnaK system (consisting of DnaK, DnaJ and GrpE), the GroEL system (consisting of GroEL and GroES), IbpB, HtpG and ClpB (final concentration: 2 µM) and an ATP regenerating system were added under various conditions and incubated for 4 hours at 30°C. Soluble and insoluble protein material was separated by centrifugation. The amount of soluble and insoluble fractions were determined by scintillation counting. As shown in Figure 4, when added individually, none of these chaperones had the potential to solubilize the aggregated proteins within a time period of 4 hours, even when added at high concentration with respect to the aggregated proteins. In contrast, when ClpB and the DnaK system were added together at 2 µM final concentration, approximately 50 % of the aggregated *E. coli* proteins were solubilized within 4 hours at 30°C (Fig. 4).

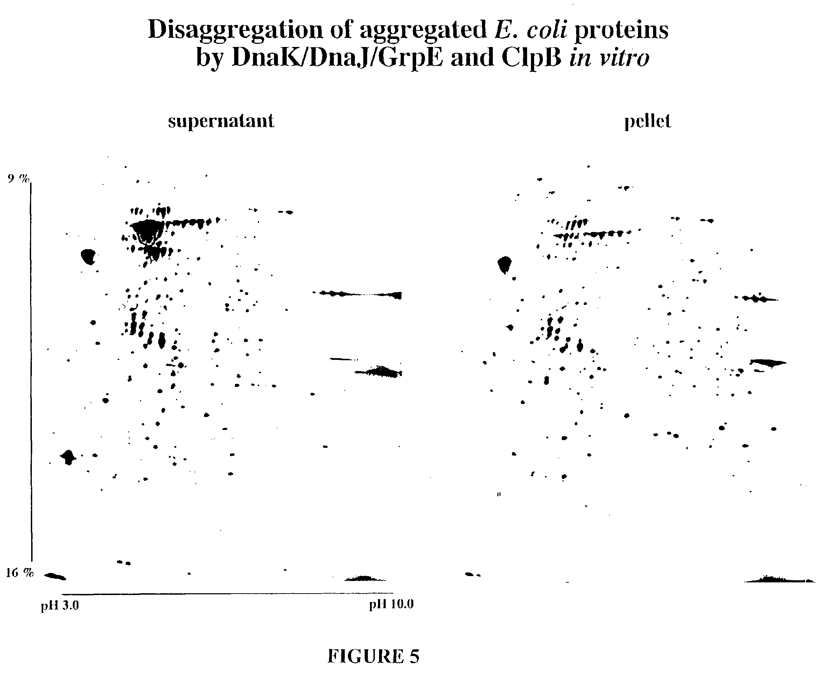

Soluble extracts of *E. coli* wild type cells were preincubated at 30°C for 5 min. and heat-shocked to 45°C for 15 min. Aggregated proteins were isolated by centrifugation and resuspended in reaction buffer (50 mM HEPES pH 7.6; 150 mM KCl; 20 mM MgCl2) by pipetting. ClpB (2 µM), and the DnaK system consisting of DnaK (2 µM), DnaJ (0.4 µM) and GrpE (0.2 µM) were added together with an ATP regenerating system. After incubation for 4 hours at 30°C, soluble and insoluble fractions were separated by centrifugation and analyzed by two-dimensional gel electrophoresis following standard protocols. As shown in Figure 5, at least 70 % of the different aggregated protein species showed increased solubility after incubation with ClpB and the DnaK system (Fig. 5).

### Example 4: The DnaK system/ClpB combination disaggregates and refolds thermosensitive proteins in vitro

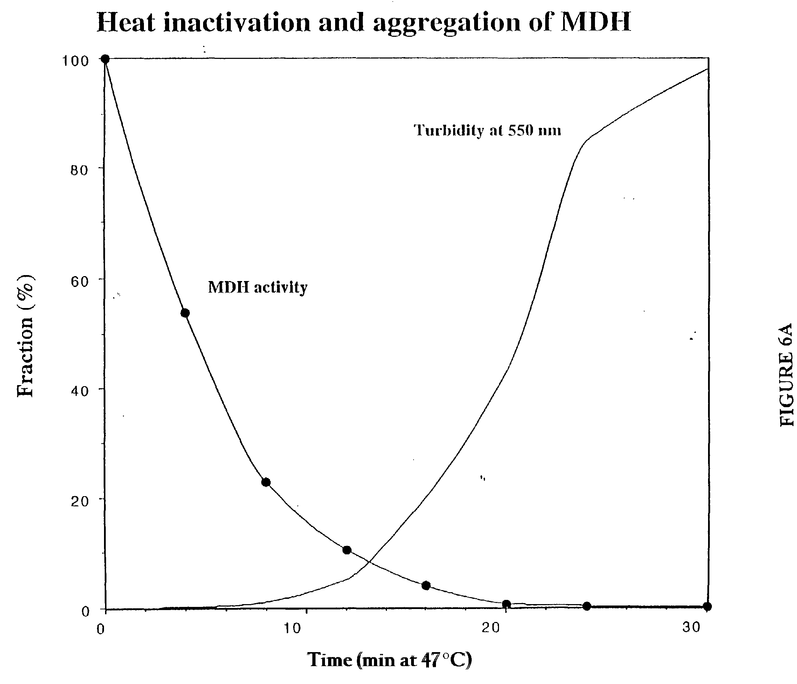

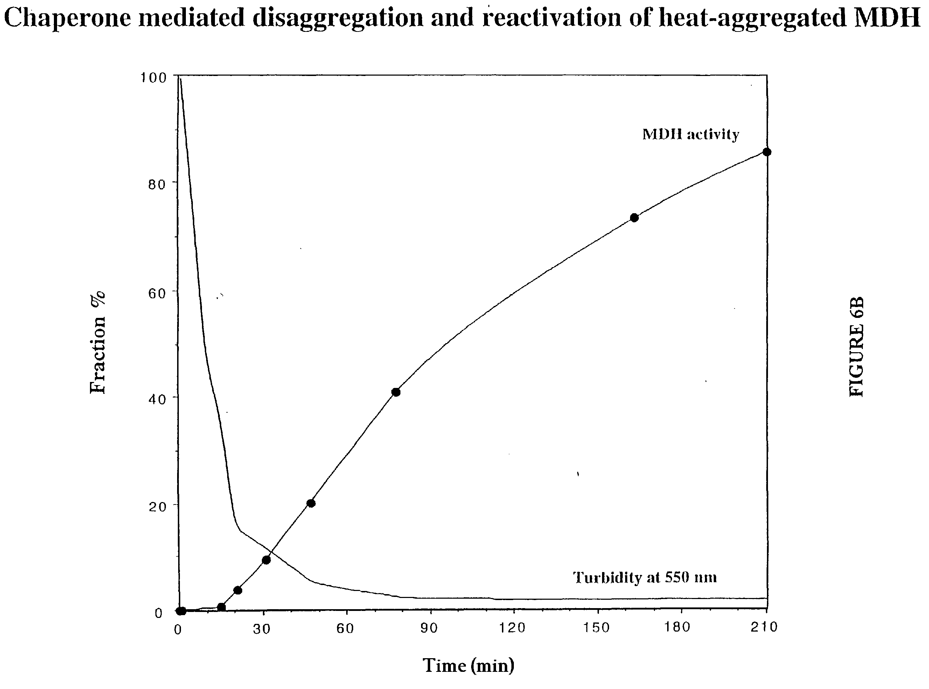

A set of experiments was conducted to analyze the ability of various chaperones to disaggregate and refold aggregates of thermosensitive test proteins (including Malate Dehydrogenase (MDH) and firefly luciferase). Qualitatively similar results were obtained for all proteins tested, and the results for MDH are summarized in Figure 6 and Table 3, and described in more detail below.

Incubation of MDH at 47°C caused inactivation and formation of large aggregates, as judged by loss of its enzymatic activity, an increase in light scattering (turbidity) of the protein solution, and microscopic detection of aggregates. This is depicted in Figure 6A which shows the time-dependent inactivation and aggregation (increased turbidity at 550 nm) of mitochondrial MDH (720 nM) at 47°C without chaperones and in the presence of DTT (10 mM). As shown in Table 3 and Figure 6A, neither ClpB nor the DnaK system alone, with or without ATP, was active in disaggregation and refolding of MDH. In contrast, as shown in Table 3 and Figure 6B (which shows the time-dependent disaggregation and reactivation at 25°C of MDH that had been aggregated by heat treatment as described above but supplemented with ClpB, DnaK, DnaJ and GrpE at concentrations of 500, 1000, 200, and 100 nM, respectively), the combined ATP-dependent activity of, ClpB and the DnaK system allowed complete solubilization within 30 min. and almost complete reactivation of up to 3 µM MDH within 3-4 hours.

**Table 3**

| Disaggregation of aggregates of Malate Dehydrogenate (MDH) by chaperones | | | | |
|---|---|---|---|---|
| Time of addition | | Rate disagg. | | Refolding yields % |
| t=0 | t=45 | nN.min.⁻¹ | | (20 hrs) |
| BKJE | | 47 | t₀ | 96 |
| B | KJE | 61 | t₄₅ | 98 |
| KJE | B | 46 | t₄₅ | 98 |
| B | J | 0 | t₄₅ | ∼3 |
| B | K (1 µM) | 0.5 | t₄₅ | ∼4 |
| B | KJE + LS | 37 | t₄₅ | 89 |
| B | hptG | 0 | t₄₅ | > 1 |
| B | HscA/B | 0 | t₄₅ | > 1 |
| KJE | | 0.3 | t₄₅ | 13 |
| B | | -4* | t₄₅ | > 1 |

Table 3 legend: Rates of disaggregation were measured either at t = 0' (to) or at t = 45' (t45). Unless indicated otherwise, the concentrations were as follows: MDH.agg, 0.72 µM; ClpB, 0.5 µM, DnaK, 1 µM; DnaJ, 0.2 µM, GrpE, 0.1 µM; GroEL, 4 µM; GroES, 4 µM; hptG, 1 µM. *ClpB and ATP caused additional aggregation rather than disaggregation. In the table, B refers to ClpB, KJE refers to the DnaK system (DnaK, DnaJ and GrpE), LS refers to GroEL-GroES, and HscA/B refers to HscA and HscB (homologs of DnaK and DnaJ, respectively).

Examples 3 and 4 demonstrate that ClpB and the DnaK system form a cooperative chaperone network that is highly efficient in solubilization and reactivation of a broad spectrum of aggregated proteins *in vitro*.

### Example 5: In vivo activity of the DnaK system-ClpB combination

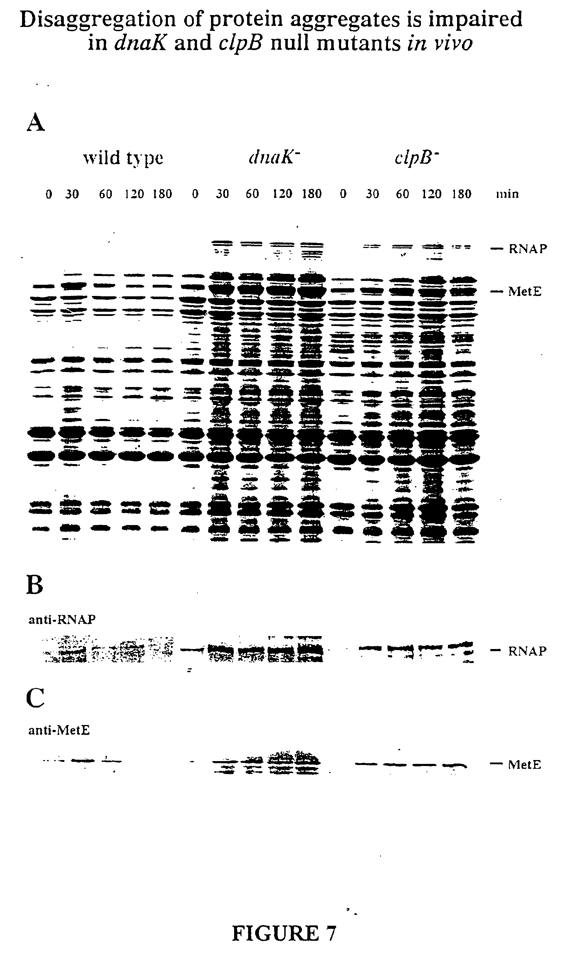

*In vivo* experiments were performed to determine whether the combination of the DnaK system and ClpB is effective in *E. coli* cells to reverse the formation of protein aggregates. The protein disaggregation potential of wild type cells was compared with that of mutant strains carrying deletions in either *clpB* (Squires et al., 1991) or *dnaK* (Paek and Walker, 1987). *E. coli* wild type, *dnaK* and *clpB* mutant cells were grown exponentially at 30°C and heat-shocked to 45°C to induce protein misfolding. Insoluble cell fractions (including membrane proteins) were prepared by centrifugation before and at the times indicated in Figure 7(A) after heat treatment. Heat-labile proteins were visualized and identified by Coomassie-stained SDS-polyacrylamide gel electrophoresis in (Fig. 7A, followed by immunoblotting (Figs. 7B and C). In wild type cells the temperature upshift caused only minor and transient accumulation of aggregated protein. In *zIdnaK* mutant cells, as outlined above in detail, a large number and amount of different proteins aggregated irreversibly. In *AclpB* mutant cells, a small amount of different proteins aggregated after temperature upshift and did not disaggregate within 3 hours as shown in Figure 7. This aggregation behavior of cellular proteins is shown in more detail in Figure 7C for subunits of the RNA polymerase and the MetE protein by using immunodetection.

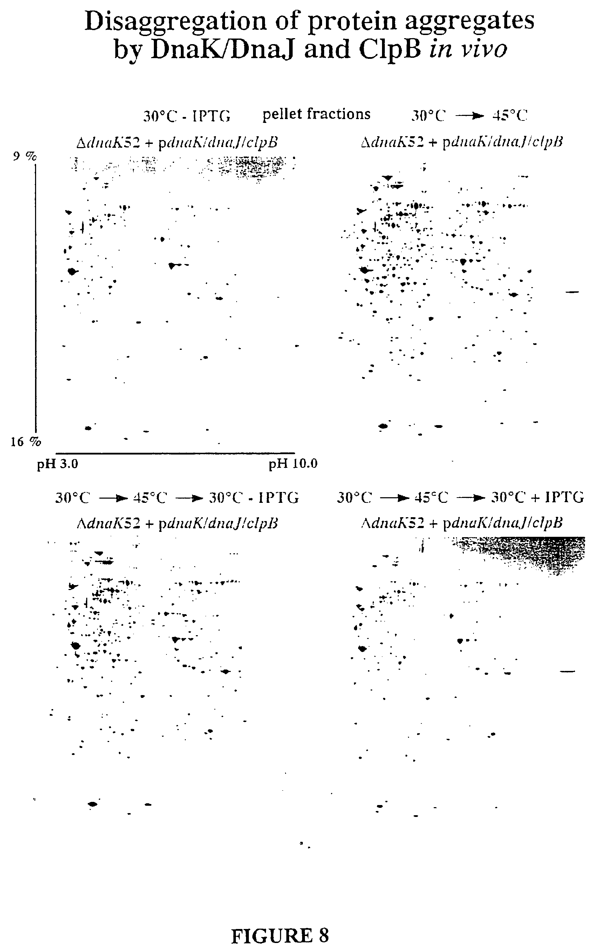

Based on these findings, *in vivo* tests were conducted to determine whether the extensive aggregation of proteins in the *ΔdnaK* mutant cells at 45°C can be reversed by subsequent overproduction of ClpB and/or the DnaK system. For this purpose a plasmid was constructed to induce expression of the respective genes by addition of IPTG into the culture medium. Accordingly, *E. coli* mutant cells which lack the *dnaK* gene *(ΔdnaK52)* and carry a plasmid allowing the overexpression of *dnaK, dnaJ* and *clpB (pdnaK/dnaJ/clpB)* were grown exponentially at 30°C. Cells were heatshocked to 45°C for 30 min. followed by re-transfer to 30°C for 2 hours. Induction of chaperone synthesis was achieved by addition of IPTG ( 1 mM). Further incubation in absence of IPTG served as negative control. Insoluble cell fractions (including membrane proteins) were prepared and analyzed by two-dimensional gel electrophoresis (Figure 8) following standard protocols. As shown in Figure 8, the induction of expression of *clpB* and *dnaK, dnaJ* and *grpE* allowed the efficient solubilization of aggregated protein, by 70 - 80 % within 2 hours at 30°C. These findings indicate that it is possible to solubilize a wide spectrum of aggregated proteins *in vivo* by cooverproducing ClpB and the DnaK system.

### Example 6: Plasmid based expression of ClpB, DnaK, DnaJ and GrpE and the GroEL-GroES chaperone system

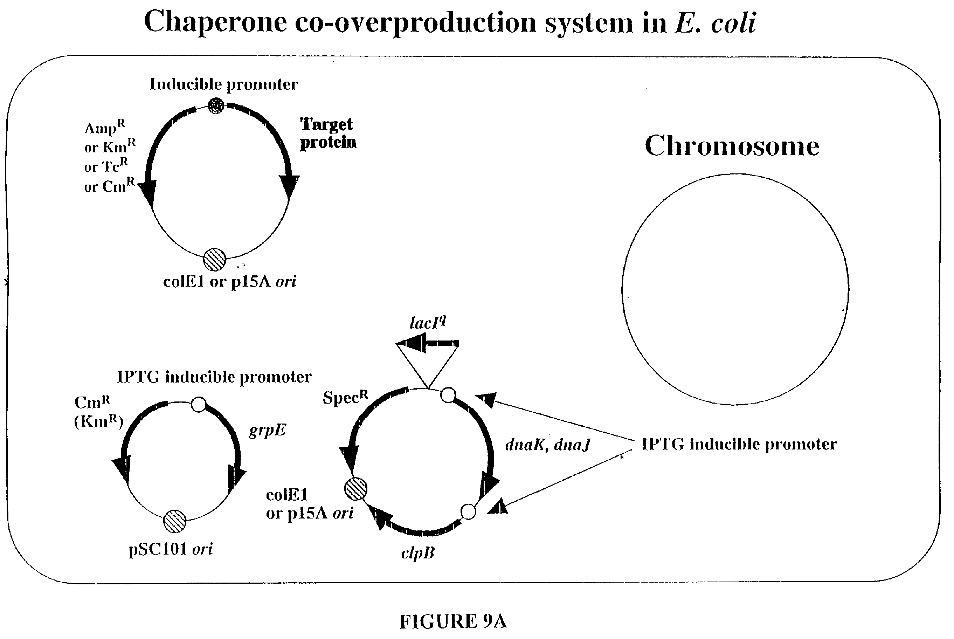

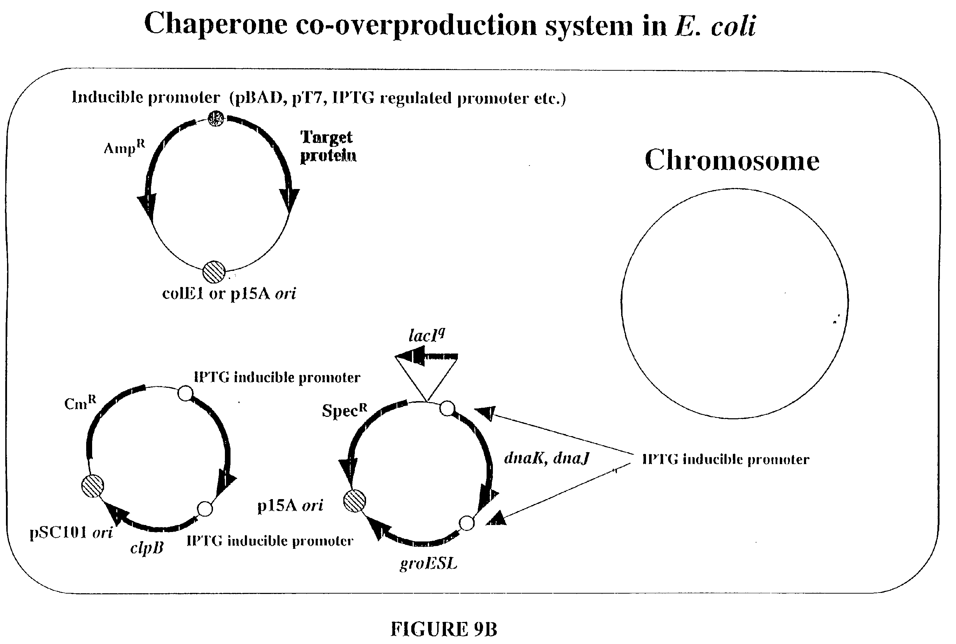

Figures 9A and 9B depict a schematic representation of plasmid-based expression that allows for the independent co-overproduction in *E. coli* of recombinant proteins together with ClpB, DnaK, DnaJ and GrpE, as well as with the GroEL-GroES chaperone system. A set of compatible plasmids was constructed allowing the independently regulated expression of genes encoding ClpB, the DnaK system, the GroEL/GroES system, and recombinant target proteins. This system is based on elements of a cassette-like plasmid system developed by Bujard and coworkers (Lutz and Bujard, 1997) and the arabinose regulatable expression vector (Mayer, 1995) with modifications.

The cassette system was used to express the chaperone genes such that the stoichiometries of the individual chaperone proteins are adjustable to their normal stoichiometries found in wild type *E. coli* cells. This adjustment provides an optimal chaperone activity (Packschies *et al.*, 1997) (unpublished observation). The cassette system allows the exchange of the origin of replication *(e.g.,* colEl, pl5A, pSC101) and, consequently, the exchange of the plasmid copy number from approx. 5/cell to 50/cell. Expression of the chaperone genes is driven by the IPTG inducible promoter Pallla (Lanzer, 1988; Lutz and Bujard, 1997). The antibiotic resistance cassette provides a choice between different resistance genes *(e.g.*,. Ap, Km, Cm, Sp). The arabinose regulatable vector allows expression of genes for recombinant target proteins from the arabinose inducible PgAD promoter (Mayer, 1995). Appropriate restriction sites allow the exchange of the resistance genes and the origin of replication according to particular uses.

The expression system of the example is not the only possibility to cooverproduce chaperones and target proteins. Many different vector systems, as well as chromosomally integrated expression systems, are possible and readily apparent to the skilled artisan.

### Example 7: A protease deficient, recombinant molecular chaperone system E. coli production strain useful to produce high levels of recombinant proteins

In order to provide high level production of recombinant proteins that are unstable in *E. coli*, *a* special production strain was designed which takes advantage of a published chromosomal deletion affecting protease production. This strain is deficient in major cytosolic proteases due to a deletion in the rpoHgene that encodes the transcriptional activator, δ³² (Kusukawa and Yura, 1988). At the same time, it produces constitutively high levels of GroEL and GroES because of transcriptional activation of the *groES groEL* operon by an insertion element. This strain was combined with the plasmid-based, regulated overexpression of *clpB, dnaK, dnaJ, grpE, groES* and *groEL* and the gene encoding recombinant protein. This combination of protease deficiency and chaperone overproduction renders this strain highly useful as a production strain in many biotechnological applications.

### Example 8: Chaperone usage in the treatment of diseases linked to protein malfunction

Chaperones are useful in preventing and reversing the aggregation of proteins linked to amyloidoses and prion diseases. Several neuro-degenerative and age related diseases, such as the Creutzfeld-Jakob and Alzheimer diseases are caused by the accumulation of misfolded proteins, in the form of amyloid plaques which are often cytotoxic. Similarly, environmental stress, such as heat shock, can induce irreversible protein aggregation leading to cell death. The foregoing examples demonstrate that a molecular chaperone system comprising a combination of *E. coli* ClpB and DnaK chaperones mediates active disaggregation and reactivation of aggregated proteins in vitro as well as *in vivo*. This novel combination provides the foundation for research may lead to characterization of a new type of intracellular defense mechanism in humans and other animals against protein misfolding and prion diseases, and against neuropathological amyloid diseases in particular. Therefore, the present invention contemplates the coupled action of the Hsp70 system and ClpB or Hsp100 cognates in humans as a design strategy for therapies for a number of diseases including, for example, prion-mediated diseases and age-related amyloid diseases.

### Example 9: Combination of Trigger Factor and the DnaK system useful for initial protein folding

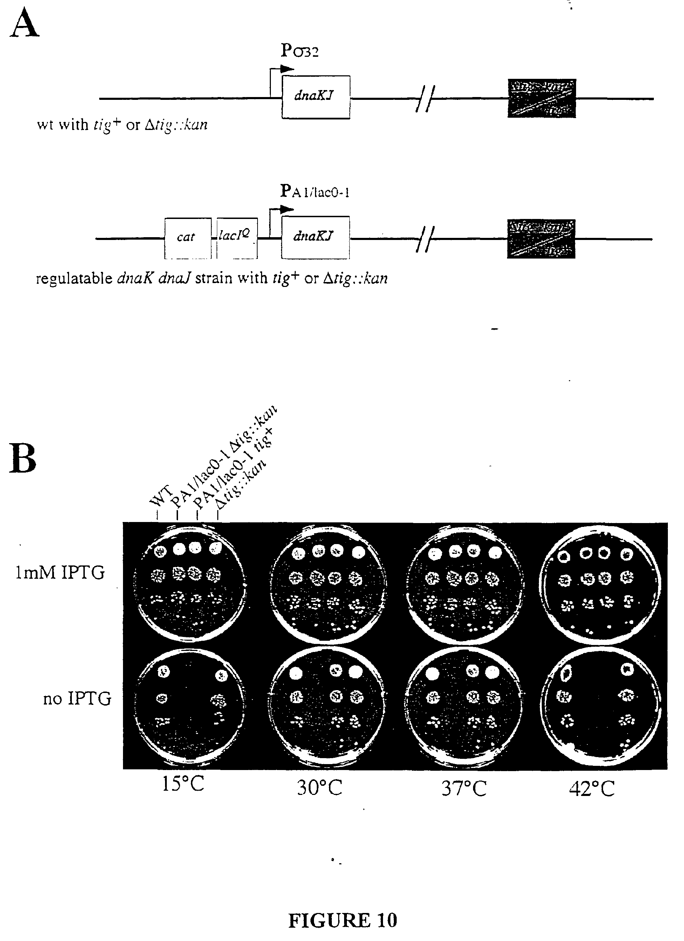

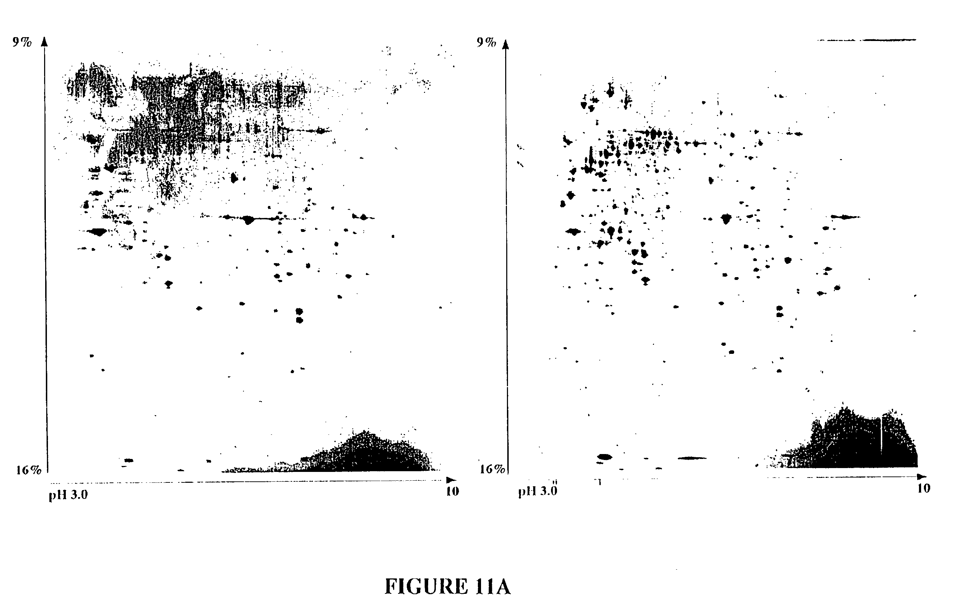

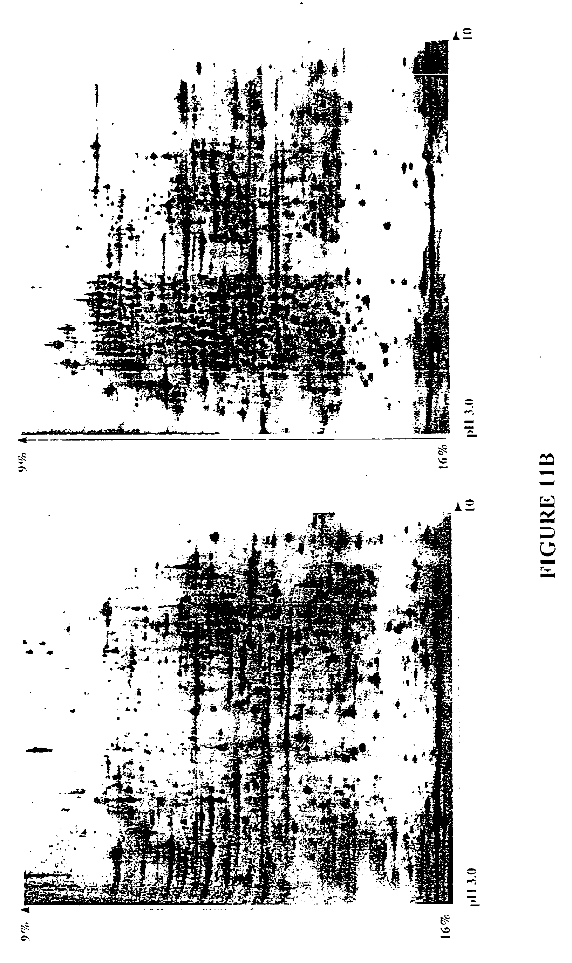

Neither the DnaK system nor the Trigger Factor is essential for initial folding of newly synthesized proteins in *E. Coli* (Hesterkamp and Bukau, 1998; unpublished data). Figure 10A is a schematic representation of the chromosomal *dnaK, dnaJ* operon regulated by its native δ³² promoter or by the artificial IPTG inducible promoter PA l/lacO in tig⁺ and *Δtig::kan* background. Wild type, *Δtig::kan* and DnaKiDnaJ regulatable *Δtig::kan* and tig⁺ cell cultures were applied in different dilutions (10⁵, 10⁴, 10³, 10² cfu/ml; 10 µl/spot) on LB plates with or without 1 mM IPTG. Cells were incubated at 30° C, 37° C and 42° C for 24 hours and at 15°C for 96 hours. Figure 10B shows that the lack of Trigger Factor and DnaK is lethal for *E. coli*. Cultures of DnaK/DnaJ depleted tig⁺ and *Δtig::kan* cells were grown at 37° C in M9 minimal media to the mid log phase (OD600 = 0.6) and pulsed with ³⁵S-methionine (15 µCi/ml final concentration) for 15 seconds. Identical amounts of total protein (1.2 ml of 2mg/ml total protein lysate) were centrifuged to isolate insoluble proteins and analyzed by two-dimensional gel electrophoresis following standard protocols (Figs. 11A and B). Using firefly luciferase (a reporter protein) as the target protein, Figure 11 demonstrates that in a strain that lacks Trigger Factor (because of deletion of the *tig* gene) and that is depleted for DnaK (because of an IPTG-regulated shut off of expression of *dnaK*, a reporter protein (firefly luciferase) has reduced folding efficiency, and a large number and high amount of cellular proteins fail to reach native state and aggregate. The combined action of both chaperone systems is thus of broad significance for folding of newly synthesized proteins in *E. coli.* Furthermore, existence of Trigger Factor and DnaK homologs in a wide variety of eubacteria suggests that cooperation between these two chaperones is of general importance for protein folding in the cytosol of prokaryotes.

This knowledge is useful to improve the folding efficiency in prokaryotes by overproducing Trigger Factor, perhaps but not necessarily together with the DnaK system. Furthermore, the addition of Trigger Factor to *in vitro* protein synthesis systems (*in vitro* translation systems), e.g., currently developed for the functional analysis of genomes, may increase the efficiency of folding of the protein products.

### Example 10: Identification of an E. coli gene encoding ClpS - a novel Hsp100 family co-chaperone

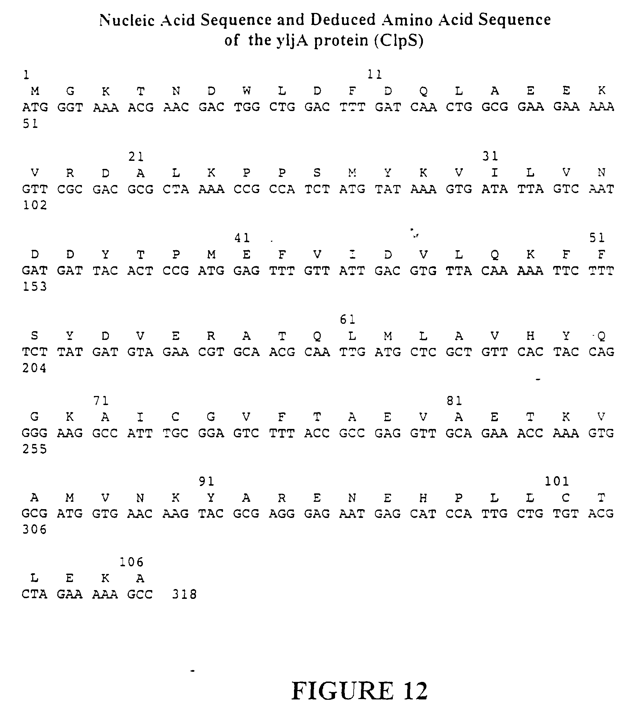

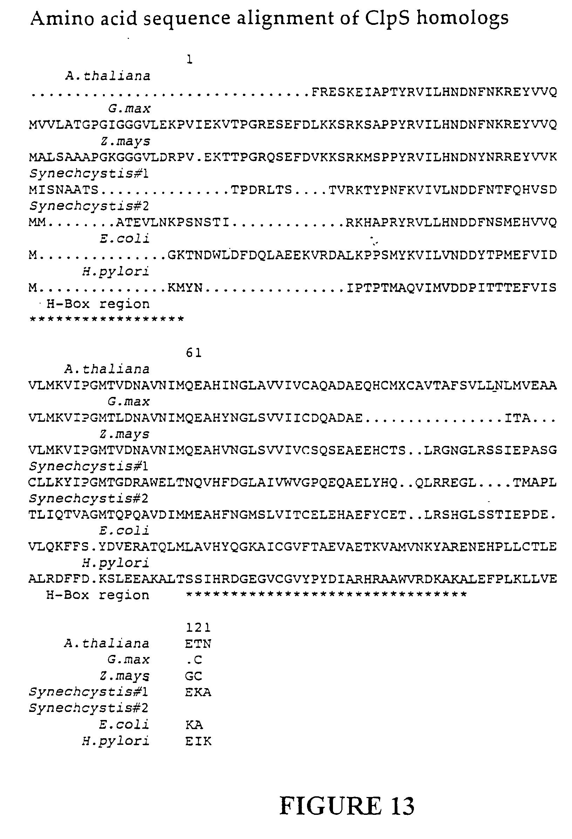

An *E. coli* gene "yljA", the first gene in the yljA-clpA operon, was cloned using standard protocols. The yljA gene is 318 bp in length and encodes a 106 amino acid protein called "ClpS". Figure 12 shows the DNA sequence of yljA (SEQ ID NO: 1) and the deduced amino acid sequence of the ClpS protein (SEQ ID NO: 2). These are identified in Table 4, and Figure 13 shows an amino acid sequence alignment of ClpS homologs. The proteins identified in Table 4 are the result of BLAST and FASTA searches and represent putative ClpS homologs. It is interesting to note that, as in *E. coli*, the *Helicobacter pylori* (HP0032) homolog occurs in an operon with the ClpA homolog, as it does in *E. coli* Despite 33% homology across the whole protein, HP0032 is believed to be a ClpS homolog based on sequence identity and position.

Tables 5 and 6 set forth, respectively, the overall sequence similarities in the ClpS homologs, and the sequence similarities in the H-BOX region (see Figure 13), a region conserved through evolution, in ClpS homologs. The sequences in Figure 13 were aligned by the "Clustal" method (Higgins and Sharp, Gene, 15:237-44, 1988) and sequence similarities in Tables 5 and 6 were calculated using the "Megalign" program (Clewley and Arnold, Methods Mol. Biol. 70:119-29, 1997).

**Table 4**

| DNA Accession Numbers for ClpS homologs | |
|---|---|
| Name | DNA Accession Number |
| *Arabidopsis thaliana* | AA586096¹ |
| *Glycine max* | AI437539¹ |
| *Zea mays* | AA979843¹ |
| *Synechcystis#1* | SSD908² |
| *Synechcystis#2* | SSD914² |
| *Helicobacter pylori* | HP0032³ |

| | |
|---|---|
| ¹ dbEST accession number | |
| ² EMBL accession number | |
| ³ GenBank accession number | |

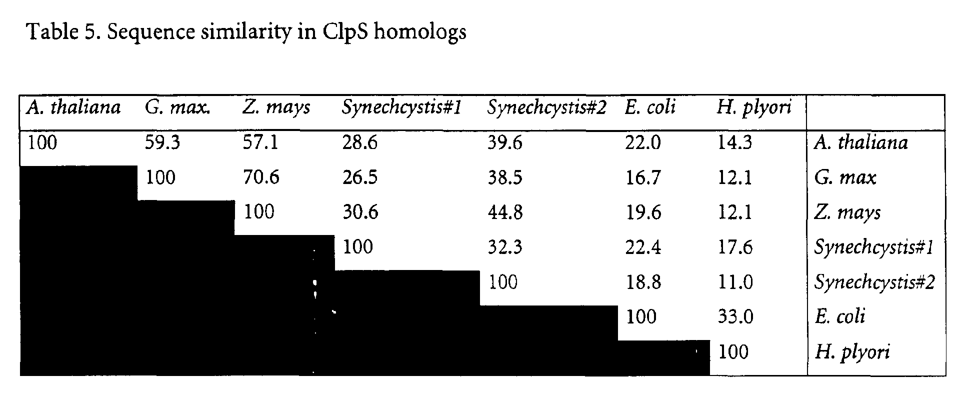

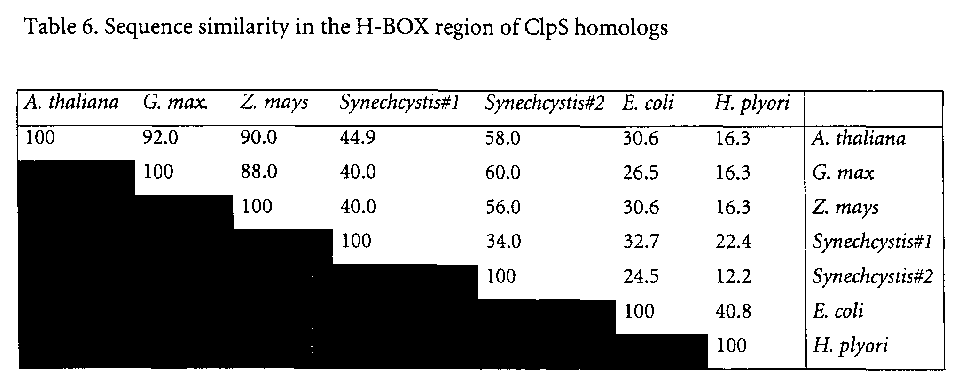

### Example 11: ClpS is established as a co-chaparone of ClpA

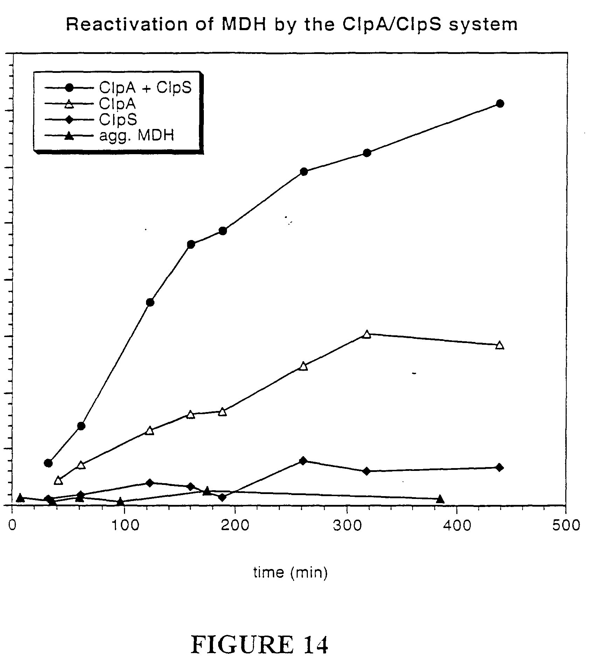

Malate dehydrogenase (MDH) (0.9 µM) was aggregated, in the absence of chaperones, by incubation at 47°C for 30 minutes. With reference to Figure 14, following aggregation, MDH activity was monitored in the absence of chaperones (filled triangle), in the presence of 0.5 µM ClpS (filled diamond), 0.5 µM ClpA (open triangle), or 0.5 µM ClpA + 0.5 µM ClpS (filled circle). As indicated in Figure 14, in the absence of chaperones or the presence of ClpS alone, MDH did not regain significant activity. In the presence of ClpA alone, up to 30% MDH activity was obtained after 300 minutes. When ClpA is supplemented with ClpS, both the rate and the yield of MDH activity was enhanced more than two-fold. Thus, ClpS is established as a potent co-chaperone of ClpA.

### Example 12: Protease deficient cells accumulate aggregated proteins under DnaK/DnaJ depleted conditions.

This experiment (Fig. 16) shows that the proteases Lon and ClpXP are crucial for elimination of protein aggregates in *E. coli* cells with low chaperone content (DnaK/DnaJ). The yields of recombinant proteins produced in *E. coli* production strains or in *in vitro* translation systems may therefore be improved by use of mutants lacking CIpXP and Lon.

A. Cells of strains BB7349 (PA1/lacO-1 *dnaK, dnaJ lacIq*), BB7353 (PA1/lacO-1 *dnaK, dnaJ lacIq hslVU*), BB7357 (PA1/lacO-1 *dnaK, dnaJ lacIq clpPX- lon*), BB7361 (PA1/lacO-1 *dnaK, dnaJ lacIq ΔhslVU clpPX - lon*) were grown at 30°C over night in LB medium with 1mM IPTG. The precultures were diluted 1000-fold in LB medium with or without 500mM IPTG and further cultured until late log phase (around OD600=1). Aggregated proteins were isolated from 8 ml of culture and membrane proteins were removed by washing with NP40. The aggregated proteins from 3.2 ml of culture/OD600 were analyzed by SDS-PAGE followed by staining with Coomassie Brilliant Blue. Aliquots of total cell lysates and aggregated protein fractions were quantified by Bradford assay. PA/lacO-1 K,J; BB7349, ΔVU; BB7353, ΔXP Ion; BB7357, ΔXP Ion ΔVU; BB7361, +; cultured in the presence 500mM IPTG, -; cultured without IPTG.

B. 1000-fold diluted cultures were further cultured until mid log phase (around OD= 0.5) in LB with several concentrations of IPTG (0, 50, 500mM) and further cultured for 2hrs at 42°C. Aggregated proteins were isolated from 8ml culture and membrane proteins were removed as above. The aggregated proteins from 1.6ml culture/OD600 were analyzed by SDS-PAGE and quantified as above. % of total proteins were calculated from the amount of aggregated protein divided by total protein amount in the cell.

### Example 13: Overproduction of Clp3 and the Dnak system improves heat resistance and luciferase refolding of rpoH and wild type cells.

This experiment (Fig. 15) shows that ClpB and the DnaK system act cooperatively in improving (i) the survival of cells (*rpoH* mutants) at lethal heat shock temperatures and (ii) the reactivation of a thermolabile reporter enzyme (firefly luciferase) in rpoH and wild type cells which were subjected to transient exposure to lethal heat shock temperatures.

A. (*ΔrpoH* cells); ClpB (*ΔrpoH* cells with ClpB overproduced from plasmids); KJE (*ΔrpoH* cells with DnaK, DnaJ, GrpE overproduced from plasmids); KJE, ClpB (Δ*rpoH* cells with DnaK, DnaJ, GrpE and ClpB overproduced from plasmids) were grown at 30C in LB medium supplemented with 250 mM IPTG which induces expression of plasmid encoded ClpB, DnaK, DnaJ, GrpE. Cells were treated at 50C at indicated time points and then plated onto LB agar plates containing 250 mM IPTG. After 48 hrs incubation, colony numbers were counted and survival rates were determined. For control nonheat treated cells were set as 100%.

B. WT (wild type cells); (*ΔrpoH* cells); ClpB *(rpoH* cells with ClpB overproduced); KJE (*ΔrpoH* cells with DnaK, DnaJ, GrpE overproduced); KJE, ClpB (*ΔrpoH* cells with DnaK, DnaJ, GrpE and ClpB overproduced) and ESL (*ΔrpoH* cells with GroESL overproduced) containing arabinose inducible luciferase were grown in LB medium supplemented 250 mM IPTG followed by induction of luciferase in the presence of arabinose (0.4%) for 30 min. Aliquots (same OD, 100 ml) were incubated for 0, 2.5 5, 7.5 min at 50°C in the presence of tetracycline (100 mg/ml) and glucose (1%), followed by further incubation for 10 min at 30°C. Recovery (%) was calculated compared to luciferase activity of noheat treated cell (set as 100%)

The foregoing description of the invention has been presented for purposes of illustration and description. The preferred embodiments described herein above are further intended to explain the best mode known of practicing the invention and to enable others skilled in the art to utilize the invention in various embodiments and with various modifications adapted to their particular applications or uses of the invention.

Further, the description is not intended to limit the invention to the form disclosed herein. Those skilled in the art will recognize, or will be able to ascertain using no more than routine experimentation, many equivalents to the specific embodiments of the invention described herein. Such equivalents are intended to be encompassed by the following claims.

### References

Buchberger, A., Schroder, H., Hesterkamp, T., Schonfeld, H.-J., and Bukau, B. (1996). Substrate shuttling between the DnaK and GroEL systems indicates a chaperone network promoting protein folding. J. Mol. Biol. *261,* 328-333.

Bukau, B. (1999). Molecular Chaperones and Folding Catalysts - Regulation, Cellular Function and Mechanisms (Amsterdam: Harwood Academic Publishers), pp. 690.

Bukau, B., and Horwich, A. L. (1998). The Hsp70 and Hsp60 Chaperone Machines. Cell 92, 351-366.

Clewley, J.P. and Arnold, C. (19977). MEGALIGN. The multiple alignment module of LASERGENE. Methods Mol. Biol., 70:119-29.

Craig, E. A., and Gross, C. A. (1991). Is hsp70 the cellular thermometer? TIBS *16*, 135-140.

Deuerling, E., Schulze-Specking, A., Tomoyasu, T., Mogk, A. and Bukau, B. (1999) Trigger factor and DnaK cooperate in folding of newly synthesized proteins. Nature, 400, 693-696.

Ehrnsperger, M., Hergersberg, C., Wienhues, U., Nichtl, A., and Buchner, J. (1998). Stabilization of Proteins and Peptides in Diagnostic Immunological Assays by the Molecular Chaperone Hsp25. Analyt. Biochem. *259*, 218-225.

Ewalt, K.L., Hendrick, J.P., Houry, W.A. and Hartl, F.U. (1997) *In vivo* observation of polypeptide flux through the bacterial chaperonin system. Cell, 90, 491-500.

Freeman, B. C., Toft, D. O., and Morimoto, R. I. (1996). Molecular chaperone machines: Chaperone activities of the cyclophilin Cyp-40 and the steroid aporeceptorassociated protein p23. Science 2 74, 1718- 1720.

Glover, J. R., and Lindquist, S. (1998). HsplO4, Hsp70, and Hsp40: A Novel Chaperone System that Rescues Previously Aggregated Proteins. Cell 94, 73-82.

Hartl, F. U. (1996). Molecular chaperones in cellular protein folding. Nature *381*, 571-580.

Hesterkamp, T., and Bukau, B. (1998). Role of the DnaK and HscA homologs of Hsp70 chaperones in protein folding in *E. coli.* EMBO J. 1 7, 4818-4828.

Higgins, D.G and Sharp, P.M. (1988). CLUSTAL: a package for performing multiple sequence alignment on a microcomputer. Gene, 73:237-44.

Horwich, A. L., and Weissman, J. S. (1997). Deadly Conformations - Protein Misfolding in Prion Disease. Cell 89, 495-510.

Jaenicke, R., and Seckler, R. (1999). Spontaneous versus assisted protein folding. In Molecular Chaperones and Folding Catalysts. Regulation, Cellular Function and Mechanism, B. Bukau, ed. (Amsterdam: Harwood Academic Publishers), pp. 407-436.

Johnson, J. L., and Craig, E. A. (1997). Protein folding *in vivo:* Unraveling complex pathways. Cell 90, 201-204.

Kusukawa, N., and Yura, T. (1988). Heat shock protein GroE of *Escherichia coli:* key protective roles against thermal stress. Genes & Dev. 2, 874-882.

Langer, T., Lu, C., Echols, H., Flanagan, J., Hayer, M. K., and Hartl, F. U. (1992). Successive action of DnaK, DnaJ and GroEL along the pathway of chaperone-mediated protein folding. Nature 356, 683-689.

Lanzer, M. (1988). Untersuchungen zur Kompetition zwischen Repressor und RNA -Polymerase um DNA - Bindungsorte: University of Heidelberg, Heidelberg, Germany).

Lilie, H., Schwarz, E., and Rudolph, R. (1998)∼ Advances in refolding of proteins produced in E. *coli.* Curr. Opin. Biotechnol. 9, 497-501.

Lindquist, S. (1997). Mad Cows Meet Psi-chotic Yeast: The Expansion of the Prion Hypothesis. Cell 89, 495-498.

Lindquist, S. (1998). Methods and compositions of genetic stress response systems. U.S. Patent 5,827,685.

Lutz, R., and Bujard, H. (1997). Independent and tight regulation of transcriptional units in *Escherichia coli* via the LacRJO, the TetR/O and AraC/Il-I2 regulatory elements. Nucleic Acids Research 25, 1203-1210.

Mayer, M. P. (1995). A new set of useful cloning and expression vectors derived from pBlueScript. Gene 163, 41-46.

Mitraki, A., and King, J. (1989). Protein Folding Intermediates and Inclusion Body Formation. BioTechnology 7, 690-697.

Morimoto, R., Tissieres, A., and Georgopoulos, C. (1994). The biology of heat shock proteins and molecular chaperones (Cold Spring Harbor, NY: Cold Spring Harbor Laboratory Press).

Packschies, L., Theyssen, H., Buchberger, A., Bukau, B., Goody, R. S., and Reinstein, J. (1997). GrpE accelerates nucleotide exchange of the molecular chaperone DnaK with an associative displacement mechanism. Biochemistry 36, 3417-3422.

Paek, K. H., and Walker, G. C. (1987). *Escherichia coli* dnaK null mutants are inviable at high temperature. J. Bact. 169, 283-290.

Parsell, D., A., Kowal, A., S., Singer, M., A., and Lindquist, S. (1994). Protein disaggregation mediated by heat-shock protein HsplO4. Nature 372, 475-478.

Prusiner, S. B. (1997). Prion Diseases and the BSE Crisis. Science 278, 245-251.

Schroder, H., Langer, T., Hard, F.-U., and Bukau, B. (1993). DnaK, DnaJ, GrpE form a cellular chaperone machinery capable of repairing heat-induced protein damage. EMBO J. *12*, 4137-4144.

Skowyra, D., Georgopoulos, C., and Zylicz, M. (1990). The E. *coli dnaK* gene product, the Hsp70 homolog, can reactivate heat-inactivated RNA polymerase in an ATP hydrolysis-dependent manner. Cell 62, 939-944.

Sogo, Kazuyoetal. (1998). Chaperoneexpressionplasmids. European Patent Application No. 98111348.3, Pubn. No. EP 0885,967 A2.

Squires, C. L., Pedersen, S., Ross, B. M., and Squires, C. (1991). ClpB is the *Escherichia coli* heat shock protein F84.1. J Bacteriol 1 73, 4254-262.

Tatsuta, T., Tomoyasu, T., Bukau, B., Kitagawa, M., Mori, H., Karata, K., and Ogura, T. (1998). Heat shock regulation in *the ftsH* null mutant of *Escherichia coli:* dissection of stability and activity control mechanisms of sigma32 *in vivo*. Mol. Microbiol. 30, 583-593.

Teter, S.A., Houry, W.A., Ang, D., Tradler, T., Rockabrand, D., Fischer, G., Blum, P., Georgopoulos, C. and Hartl, F.U. (1999) Polypeptide flux through bacterial Hsp70: DnaK cooperates with Trigger Factor in chaperoning nascent chains. Cell, 97, 755-765.

Thomas, P. J., Qu,-B.-H., and Pedersen, P. L. (1995). Defective protein folding as a basis of human disease. TIBS *20*, 456-459.

Thomas, J.G. and Baneyx, F. (1998). Roles of the Escherichia coli Small Heat Shock Proteins IbpA and IbpB in Thermal Stress Management: Comparison with ClpA, ClpB, and HtpG *In vivo*. J. Bacteriology 180(19):5165-5172.

Tomoyasu, T., Ogura, T., Tatsuta, T., and Bukau, B. (1998). Levels of DnaK and DnaJ provide tight control of heat shock gene expression and protein repair in E. *coli.* Mol. Microbiol. 30, 567-581.

Turnell, W. G., and Finch, J. T. (1992). Binding of the dye congo red to the amyloid protein pig insulin reveals a novel homology amongst amyloid-forming peptide sequences. J. Mol. Biol. 227, 1205-1223.

Veinger, L., Diamant, S., Buchner, J., and Goloubinoff, P. (1998). The Small Heatshock Protein IbpB from *Escherichia coli* Stabilizes Stress-denatured Proteins for Subsequent Refolding by a Multichaperone Network. J. Biol. Chem. 273, 11032-11037.

Wetzel, R. (1996). For protein misassembly, its the "I" decade. Cell 86, 699-702.

Ziemienowicz, A., Skowyra, D., Zeilstra-Ryalls, J., Fayet, O., Georgopoulos, C., and Zylicz, M. (1993). Both the *Escherichia coli* Chaperone Systems GroEL/Gro/ES and DnaK/DnaJ/GrpE, can activate heat treated RNA Polymerase. Different mechanisms for the same activity. J. Biol. Chem. 268, 25425-25431.

## Claims

**1.** A method for regulating the conformation of a target protein comprising contacting the target protein with a molecular chaperone system comprising a first protein selected from the group comprising at least one Hsp100 protein or a homolog thereof, and a second protein selected from the group comprising at least one Hsp70 protein or a homolog thereof, to thereby regulate the conformation of the target protein.

**2.** The method of claim 1, wherein the first protein comprises a prokaryotic homolog of 10 the Hsp100 protein.

**3.** The method of claim 2, wherein the homolog is a Clp homolog.

**4.** The method of claim 3, wherein the Clp homolog is ClpA, ClpB or ClpX.

**5.** The method of claim 4, wherein the Clp homolog is ClpB.

**6.** The method of claim 1, wherein the second protein is a prokaryotic homolog of the Hsp70 protein.

**7.** The method of claim 6, wherein the prokaryotic homolog comprises DnaK.

**8.** The method of claim 1, wherein the molecular chaperone system further comprises at least one cochaperone.

**9.** The method of claim 8, wherein the cochaperone comprises DnaJ, GrpE or ClpS.

**10.** The method of claim 9, wherein the cochaperone comprises DnaJ and GrpE.

**11.** The method of claim 1, wherein the target protein is recombinantly expressed in a host cell.

**12.** The method of claim 11, wherein the target protein is aggregated due to overexpression in the host cell.

**13.** The method of claim 11, wherein the first and second proteins are recombinantly expressed in the host cell.

**14.** The method of claim 11, wherein the cell is an E. *Coli, S. Cerevisiae, B. Subtilis*, plant, insect, yeast, or mammalian cell.

**15.** The method of claim 13, wherein the first and second proteins are recombinantly expressed by transforming or transfecting the host cell with a vector encoding the first and second proteins.

**16.** The method of claim 15, wherein the vector is further capable of recombinantly expressing a GroEL-GroES system or a homolog thereof; a GroEL-GroES system protein or a homolog thereof, or Trigger Factor or a homolog thereof.

**17.** The method of claim 1, wherein the target protein is aggregated.

**18.** The method of claim 7, wherein the target protein is disaggregated *in vitro*.

**19.** The method of claim 18, wherein the target protein is aggregated due to heat stress, oxidative stress, overexpression, mutation or disease.

**20.** A recombinant expression vector which encodes at least one Hsp100 protein or homolog thereof and at least one Hsp70 protein or homolog thereof.

**21.** The vector of claim 20 which is an inducible recombinant expression vector.

**22.** The vector of claim 20 which further encodes at least one Hsp70 protein cochaperone.

**23.** The vector of claim 20 which further encodes a protein of interest.

**24.** A cell transformed with the vector of claim 20.

**25.** The cell of claim 24, wherein the cell is protease deficient.

**26.** The cell of claim 25, wherein the protease deficiency is caused by a mutation in an rpoHgene.

**27.** The cell of claim 26, wherein the mutation is a chromosomal deletion in the *rpoH* gene.

**28.** The cell of claim 24, wherein the cell, recombinantly expresses a protein of interest.

**29.** The cell of claim 28, wherein the protein of interest is likely to misfold or aggregate without recombinant expression of at least one Hsp100 protein or homolog thereof and at least one Hsp70 protein or homolog thereof.

**30.** The cell of claim 29, wherein expression of at least one Hsp100 protein or homolog thereof and at least one Hsp70 protein or homolog thereof inhibits misfolding or aggregation of the protein of interest.

**31.** The cell of claim 30, wherein the inhibition of the aggregation of the protein of interest increases the yield of the active conformation of the protein of interest compared to a cell in which at least one Hsp100 protein or homolog thereof and at least one Hsp70 protein or homolog thereof is not recombinantly expressed.

**32.** The cell of claim 22, wherein expression of at least one Hsp100 protein or homolog thereof and at least one Hsp70 protein or homolog thereof causes the disaggregation and refolding an aggregated protein.

**33.** The cell of claim 24, wherein the cell is prokaryotic or eukaryotic.

**34.** The cell of claim 33, wherein the cell is an E. *Coli, S. Cerevisiae, B. Subtilis*, plant, insect, yeast, or mammalian cell.

**35.** The cell of claim 28, wherein the protein of interest is Malate Dehydrogenase.

**36.** A method for disaggregating an aggregated protein in *vitro* comprising contacting the aggregated protein with a molecular chaperone system comprising at least one Hsp100 protein or a homolog thereof and at least one Hsp70 protein or a homolog thereof to thereby disaggregate the aggregated protein.

**37.** The method of claim 36, wherein the method further comprises the step of folding the disaggregated protein into an active form.

**38.** A method for increasing the solubility of a recombinantly expressed protein comprising coexpressing a molecular chaperone system comprising at least one Hsp100 protein or a homolog thereof and at least one Hsp70 protein or a homolog thereof to thereby increase the solubility of the recombinantly expressed protein.

**39.** A method of preventing cell death caused by misfolding and aggregation of a protein comprising administering a therapeutically effective amount of a molecular chaperone system comprising at least one Hsp100 protein or a homolog thereof and at least one Hsp70 protein or a homolog thereof to prevent the misfolding and aggregating of a protein, thereby preventing cell death.

**40.** A method of treating a disease in an animal caused by misfolding and aggregation of a protein comprising administering a therapeutically effective dose of a molecular chaperone system comprising at least one Hsp100 protein or a homolog thereof and at least one Hsp70 protein or a homolog thereof to thereby treat the disease.

**41.** The method of claim 40, wherein the animal is a human, a rodent, a cat, or a dog.

**42.** The method of claim 40' wherein the disease is Creutzfeld-Jacob's disease, Alzheimer's disease, Huntington's disease, Ataxia type-1, cystic fibrosis or cancer.

**43.** The method of claim40, wherein the dose comprises a pharmaceutically acceptable carrier.

**44.** The method of claim 43, wherein the pharmaceutically acceptable carrier is capable of targeting a misfolded protein.

**45.** A pharmaceutical composition comprising an effective dose of a molecular chaperone system comprising at least one Hsp100 protein or a homolog thereof and at least one Hsp70 protein or a homolog thereof and a pharmaceutically acceptable carrier.

**46.** A method of increasing the folding efficiency of a protein in *vitro* comprising adding Trigger Factor to an *in* VitYo protein synthesis system thereby increasing the folding efficiency of a protein *in vitro*.

**47.** The method of claim 46, further comprising adding a molecular chaperone system comprising at least one Hsp70 protein or a homolog thereof to the in vitro synthesis system.

**48.** A method of increasing the folding efficiency of a protein in a cell comprising recombinantly expressing Trigger Factor in the cell to thereby increase the folding efficiency of the protein in the cell.

**49.** The cell of claim 48, wherein the cell is prokaryotic or eukaryotic.

**50.** The method of claim 48, further comprising recombinantly expressing a molecular chaperone system comprising at least one Hsp70 protein or a homolog thereof.

**51.** An isolated polynucleic acid molecule selected from the group consisting of:
(a) a polynucleotide comprising the nucleotide sequence of SEQ ID NO:1;
(b) a polynucleotide that is antisense to the polynucleotide of (a);
(c) a polynucleotide having 80% identity to the polynucleotide of (a) or (b);
(d) a polynucleotide encoding the protein of SEQ ID NO:2;
(e) a polynucleotide encoding a polypeptide having 80% identity to the protein of SEQ ID NO:2;
(f) a polynucleotide having 80% identity to the polynucleotide of (a)-(e) encoding a protein capable of regulating the activity of Hsp100 protein or a homolog thereof in disaggregation; and
(g) a polynucleotide that hybridizes under stringent conditions to any one of the polynucleotides specified in (a)-(f)

**52.** The polynucleotide of claim 51, comprising the polynucleotide of (a).

**53.** The polynucleotide of claim 51, comprising the polynucleotide of (b).

**54.** The polynucleotide of claim 51, comprising the polynucleotide of (c).

**55.** The polynucleotide of claim 51, comprising the polynucleotide of (d).

**56.** The polynucleotide of claim 51, comprising the polynucleotide of (e).

**57.** The polynucleotide of claim 51, comprising the polynucleotide of (f).

**59.** The polynucleotide of claim 51, comprising the polynucleotide of (g).

**60.** An isolated polypeptide selected from the group consisting of:
(a) a polypeptide comprising the amino acid sequence of SEQ ID NO:2;
(b) a polypeptide having 80% identity to the amino acid sequence of SEQ ID NO:2;
(c) a polypeptide variant of the amino acid sequence of SEQ ID NO:2;
(d) a polypeptide variant of the amino acid sequence of SEQ ID NO:2 capable of regulating the activity of a Hsp100 protein or a homolog thereof in disaggregation;
(e) a polypeptide variant of the amino acid sequence of SEQ ID NO:2 capable of cochaperoning ClpA;
(f) a polypeptide encoded by a polynucleotide with the sequence of SEQ ID NO:1;
(g) a polypeptide encoded by a polynucleotide having 80% identity to the sequence of SEQ ID NO:1; and
(h) a polypeptide encoded by a polynucleotide which hybridizes under stringent conditions to a polynucleotide with the sequence of SEQ ID NO: 1.

**61.** The polypeptide of claim 60, comprising the polypeptide of (a).

**62.** The polypeptide of claim 60, comprising the polypeptide of (b).

**63.** The polypeptide of claim 60, comprising the polypeptide of (c)

**64.** The polypeptide of claim 60, comprising the polypeptide of (d).

**65.** The polypeptide of claim 60, comprising the polypeptide of (e).

**66.** The polypeptide of claim 60, comprising the polypeptide of (f).

**67.** The polypeptide of claim 60, comprising the polypeptide of (g).

**68.** The polypeptide of claim 63, wherein the variant is an insertion, a deletion, or a substitution variant.

**69.** A gene therapy for treating a disease comprising administering a pharmaceutical composition comprising a gene therapy vector encoding a molecular chaperone system comprising a first protein selected from the group consisting of at least one Hsp100 protein or a homolog thereof, and a second protein selected from the group consisting of at least one Hsp70 protein or a homolog thereof.

**70.** A composition for the gene therapy of claim 69, comprising a gene therapy vector encoding a first protein selected from the group consisting of at least one Hsp100 protein or a homolog thereof, and a second protein selected from the group consisting of at least one Hsp70 protein or a homolog thereof.

**71.** An isolated molecular chaperone system comprising a first protein selected from the group consisting of at least one Hsp100 protein or a homolog thereof, and a second protein selected from the group consisting of at least one Hsp70 protein or a homolog thereof.

**72.** A method for regulating the activity of a Hsp100 protein or a homolog thereof comprising contacting the Hsp100 protein with a polypeptide of claim 60.

**73.** The method of claim 72, wherein the Hsp100 protein is ClpA, ClpB, or ClpX.

**74.** The method of claim 73, wherein the protein is ClpA.
